(19) Europäisches Patentamt
European Patent Office
Office européen des brevets

(11) **EP 4 375 354 A1**

(12) **EUROPEAN PATENT APPLICATION**

(43) Date of publication:
**29.05.2024 Bulletin 2024/22**

(21) Application number: **23174146.3**

(22) Date of filing: **18.05.2023**

(51) International Patent Classification (IPC):
**C12G 1/02** *(2006.01)* **C12G 3/02** *(2019.01)*
**C12G 3/021** *(2019.01)* **G01N 33/14** *(2006.01)*
**C12C 11/00** *(2006.01)*

(52) Cooperative Patent Classification (CPC):
**C12G 1/02; C12C 11/003; C12C 11/006;
C12G 3/02; C12G 3/021; G01N 33/146**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.11.2022 MX 2022014836**

(71) Applicants:
• **Ibarra Hernández, Eduardo Benjamín**
  **59300 La Piedad (Michoacán) (MX)**
• **Martinez Vera, José Martín**
  **59300 La Piedad (Michoacán) (MX)**
• **Salazar Martinez, Jorge Luis**
  **59300 La Piedad (Michoacán) (MX)**

• **Plascenia Mora, Héctor**
  **59300 La Piedad (Michoacán) (MX)**

(72) Inventors:
• **Ibarra Hernández, Eduardo Benjamín**
  **59300 La Piedad (Michoacán) (MX)**
• **Martinez Vera, José Martín**
  **59300 La Piedad (Michoacán) (MX)**
• **Salazar Martinez, Jorge Luis**
  **59300 La Piedad (Michoacán) (MX)**
• **Plascenia Mora, Héctor**
  **59300 La Piedad (Michoacán) (MX)**

(74) Representative: **Torner, Juncosa I Associats, SL
C / Pau Claris, 108, 1r 1a
08009 Barcelona (ES)**

(54) **A METHOD AND A SYSTEM FOR MONITORING THE FERMENTATION OF ALCOHOLIC BEVERAGES**

(57)     A method and a system for monitoring the fermentation of alcoholic beverages are proposed. The method comprises carrying out an alcoholic fermentation process of a must to be fermented inside a fermentation tank (1); acquiring, by a plurality of sensors (2-5) arranged within the fermentation tank (1), different data of the must, said acquired data comprising: level data, pressure, temperature, and pH; receiving, by a computing device (6), the acquired data and computing a density of the must by implementing the following equation using the acquired data: $\rho = \dfrac{P}{gh}$ , where $\rho$ is the density; P is the pressure; g is the specific gravity; and h is the height of the must; and computing, by the computing device (6), the Degrees Brix of the must as a function of the computed density.

**Fig. 1**

EP 4 375 354 A1

**Description**

Technical Field

The present invention refers to the technical field of methods and systems for monitoring the fermentation processes of alcoholic beverages, in which various sensors for the monitoring of critical points are used.

Background of the Invention

The use of diverse sensors for the monitoring of alcoholic fermentations has been done for many years, particularly in the winemaking field.

However, none of the developed systems achieves the results in real time and in conditions where the must is dark; so the present invention manages to solve this problem.

Some of the documents related to the previous state of the art, describe the monitoring of alcoholic fermentation processes which are the following:

US patent application number 2012/0269925A1 describes an automatic winemaking system, that controls the execution of a winemaking process for the alcoholic fermentation of the must obtained from a batch of grapes and the transformation of said wine in a winemaking tank. The system is provided with a database to store records related to the referenced winemaking process; a first processing unit to generate an optimized winemaking model, according to the winemaking data contained in the database, according to the input data that include characteristics of the batch of grapes and/or the must; and a second processing unit to control and start the actuators that operate the winemaking tank according to the optimized winemaking model, so that the parameters of the winemaking process are optimized for the characteristics of the batch of grapes and/or the must.

International patent application WO 2020/012459A1, which mentions a device configured to undertake an on-site non-invasive control, in real time of the fermentation processes in a remote location from the fermenter/bioreactor in a laboratory or industrial environment. In which the device is configured to run high resolution online measurements that allow real time monitoring, control and optimization of the production processes based on fermentation in all scales or types of fermenters.

The device described in the international patent application WO 2020/012459A1 comprises: a detection module and light source (LSDM); an exhaust pipe adapter (ETA), that is coupled or inserted in an exhaust pipe of the fermenter/bioreactor, and it is screwed to a front face of the LDSM; and a display and control module that is communicated with the LSDM through a wired or wireless communication channel.

European patent application EP 3763828A1, describes a method for the follow up of fermentation processes, as well as a method to predict the fermentation processes and a method for the regulation of fermentation processes, furthermore it is provided with a computer program for the execution of the method as well as a device that comprises the same and a monitoring system to predict or regulate de fermentation process.

The method for the monitoring of fermentation processes includes the following stages: a) obtain a sample of the fermentation process; b) determine a pattern that includes a metagenomic marker; c) compare the pattern that includes the metagenomic marker with the pattern which comprises the metagenomic marker of a database and d) correlate the comparison in stage c) with the fermentation process in course and predict the result of the fermentation process.

US application number 2020/0292501 A1, refers to a system to detect one or more characteristics of a fluid, where the system comprises: a sonic sensor which at the same time includes: a probe body with a transduction surface and a transductor acoustically connected to the transduction surface; an acoustically reflective cushion member and a stem that has: a first end that is connected to the acoustically reflective cushion member, and a second end that is connected to the transduction surface.

The system also includes a processor and a memory that comprises programming instructions configured for the processor to generate a signal that will make the transductor to generate a set of pulses that will be transmitted to the cushion member through a fluid when the transduction surface and the cushion member are submerged in the fluid and receive the signals that indicate when the pulses have been reflected from the cushion member.

As mentioned before, none of the prior art documents specifies that the alcoholic fermentation monitoring systems perform the fermentation of dark and/or murky liquids. Moreover, none of the prior art documents performs the Degrees Brix measurement.

Summary of the Invention

An object of the present invention is to provide an integrated measurement and calculation method/system that allows monitoring the critical variables of an alcoholic fermentation process in real time, being those variables the pH, the temperature, pressure, etc. and the further calculation of the Degrees Brix, and also the fermentation efficiency and the alcohol volume percentage, of the product to be fermented.

To that end, according to a first aspect there is provided a method for monitoring the fermentation of alcoholic beverages. The method comprises carrying out an alcoholic fermentation process of a must to be fermented inside a fermentation tank; acquiring, by a plurality of sensors arranged within the fermentation tank, different data of the must, said acquired data comprising level data, pressure, temperature, and pH; receiving, by a computing device, the acquired data and computing a density of the must by implementing the following equation using the acquired data: $\rho = \dfrac{P}{gh}$ , where $\rho$ is the density; $P$ is the pressure; $g$ is the specific gravity; and $h$ is the height of the must; and computing, by the computing device, the Degrees Brix of the must as a function of the computed density.

According to a second aspect there is also provided a system for monitoring the fermentation of alcoholic beverages. The system comprises a fermentation tank at which an alcoholic fermentation process of a must to be fermented takes place; a plurality of sensors arranged within said tank to acquire different data of the alcoholic fermentation process by making direct contact with the must, said plurality of sensors comprising a radar sensor, a temperature sensor; a pH sensor and a pressure sensor, and said acquired data comprising: level data, pressure, temperature, and pH; and a computing device, configured to receive the acquired data, compute a density of the must using the received data, and to compute the Degrees Brix of the must as a function of the computed density.

In a particular embodiment, the computing device further comprises: computing the alcohol volume percentage of the must using the computed Degrees Brix; and computing the fermentation efficiency percentage of the must using the computed alcohol volume percentage.

In some embodiments, the must to be fermented is of dark color and/or is murky.

In some embodiments, the must to be fermented is selected from barley, molasses, grape juice or agave juice, among others.

In some embodiments, the computed Degrees Brix and/or the computed fermentation efficiency and/or the computed alcohol volume percentage is/are displayed on a user interface and/or on a computer application operatively connected to the computed device.

In some embodiments, the computed Degrees Brix and/or the computed fermentation efficiency and/or the computed alcohol volume percentage is/are stored in a cloud computing structure.

In some embodiments, a warning signal or an alarm is raised when the computed Degrees Brix, the fermentation efficiency and/or the alcohol volume percentage is/are outside a given permissible/desired range.

In some embodiments, the plurality of sensors are arranged at different positions within the fermentation tank.

Thus, present invention provides a monitoring system and method in which different variables (e.g. pH, temperature, hydrostatic pressure and level/height) of the must to be fermented are acquired/obtained/measured, and based on these different variables the density, Degrees Brix, fermentation efficiency and alcohol volume percentage can be calculated.

Moreover, visualization and register of the different data is enabled via a computer application. Such that a user can observe the behavior of the monitoring process in real time and also if the measured parameters are within established ranges.

Some of the main advantages of the present invention are:

- It can be used in any type of alcoholic fermentation process.
- The monitoring process is automated and can be controlled remotely (online).
- It is cost competitive compared to other known fermentation monitoring systems.
- Remote data access from anywhere in the world following the security protocols (global access).
- Data analysis for statistics and reports.
- Brix measurement using the density of the liquid/product to be fermented.

Brief Description of the Drawings

The previous and other advantages and features will be more fully understood from the following detailed description of embodiments, with reference to the attached figures, which must be considered in an illustrative and non-limiting manner, in which:

Fig. 1 is a schematic illustration of the proposed system for monitoring the fermentation of alcoholic beverages, according to an embodiment of the present invention.

Fig. 2A refers to test 1 of agave juice in one-hour intervals; comparison of the brix system using PLC vs. brix hydrometer.

Fig. 2B refers to test 2 of agave juice in two-hour intervals; comparison of the Brix system using PLC vs. Brix hydrometer.

Fig. 3A refers to test 1 of malt in two-hour intervals; comparison of the Brix system using PLC vs. Brix hydrometer.

Fig. 3B refers to test 2 of malt in two-hour intervals; comparison of Brix system using PLC vs.

Brix hydrometer.

Fig. 4A refers to test 1 of molasses in two-hour intervals; comparison of the Brix system using PLC vs. Brix hydrometer.

Fig. 4B refers to test 2 of molasses in two-hour intervals; comparison of the Brix system using PLC vs. Brix hydrometer.

Fig. 5 refers to test 1 of grape juice in two-hour intervals; comparison of the Brix system using PLC vs. Brix hydrometer.

Detailed Description of the Invention

The present invention provides a method and an integrated monitoring system for the fermentation of alcoholic beverages. The measurements/data obtained/acquired by different sensors are preferably conditioned and transmitted to a computing device, in particular, a Programmable Logic Controller (PLC).

When the signals are received by the PLC, they are processed and escalated to the actual measurement values (pH, temperature, level/heigh, and pressure). Is thus when the calculations and algorithms are executed/implemented by the PLC to obtain the value of the indirect variables. That is the density and from this parameter the Degrees Brix, fermentation efficiency and alcohol volume percentage.

On-site visualization of the data obtained can be performed on a user interface, such as a Human Machine interface (HMI). To achieve a global view, the different information is sent to a server through a modem, and in the server a data communication structure is executed allowing the transmission of information into the cloud. Once the information is stored in the cloud, queries thereof can be made via an Application Programming Interface (API); obtaining and understandable programming format for the development environment and allowing the visualization of the monitored information.

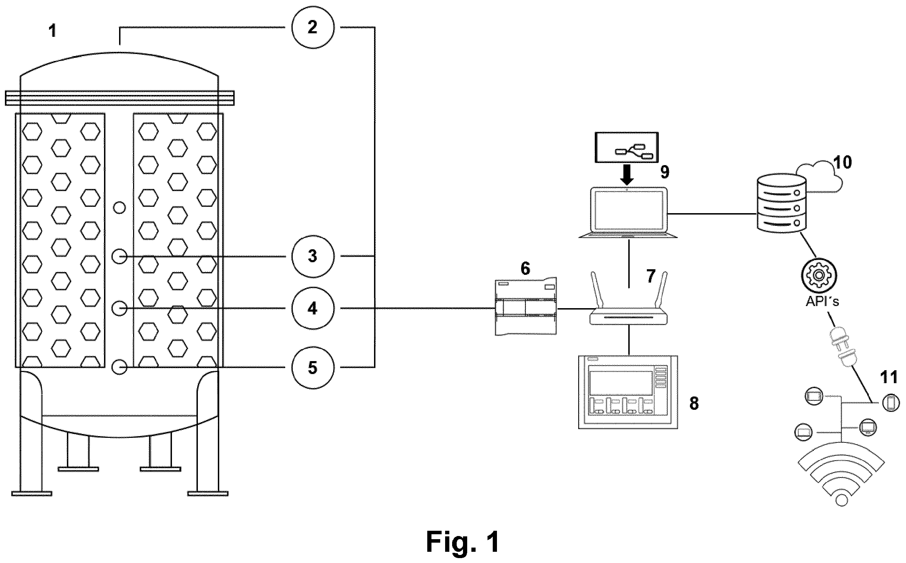

With reference to Fig. 1, an embodiment of the proposed system is shown. According to this embodiment, the system has a fermenting tank (1) in which the alcoholic fermentation process is carried out. The fermenting tank (1) comprises a radar sensor (2), particularly located at the top the tank; a RTD temperature sensor (3), particularly located one quarter down from the top of the tank; a pH sensor (4), particularly located in the middle of the tank; and a pressure sensor (5), particularly located one quarter up from the bottom of the tank. The measurement(s) of each sensor is/are conditioned and transmitted to the PLC (6). The signals are processed by the PLC (6) since it is there where the density calculations are made to later obtain the other indirect variables. The PLC (6) has communication with a modem with access to the Internet (7) to display the processed data, and said information is visualized locally in the HMI interface (8). To achieve a global view the PLC (6) also has connection to a local server (9) and in the server a data communication structure is executed allowing the transmission of data to the cloud (10); once the data is in the cloud (10) the queries of stored information are made by API's and then an understandable format is obtained for the development environments and display of the monitoring information in a computer application (11).

To develop the proposed system it is extremely important to select the sensors to be used, which will make the necessary measurements/readings to obtain level data, pressure, temperature and pH of the must (i.e. the liquid or product to be fermented). The next step is the programming and integration of the different sensors (2-4) inside the fermenting tank (1), regardless of the starting product: barley, agave, grape, among others.

Particularly, the measurements/readings are made at an 8 msec speed, in real time.

The values of each sensor (pH, temperature, level, pressure), and of the calculated variables (Degrees Brix, fermentation efficiency, alcohol volume percentage), will depend on the raw product/liquid to be fermented, as shown below in the four examples provided.

**Specifications of the process**

In an embodiment, with the measurements obtained by the level sensor in conjunction with the pressure sensor the density of the must ($kg/m^3$) is calculated using the following mathematical formula:

$$\rho = \frac{P}{gh}$$

where: $\rho$ (density); P (Pressure); g (Specific gravity); h (Height of the must).

After having computed the density of the must, the Degrees Brix of the must can be also determined, and so the fermentation efficiency and the alcohol volume percentage.

To compute the Degrees Brix, the following formulas can be used, depending on the density range:

| FORMULAS | Density range (kg/m$^3$) |
|---|---|
| Brix Polynomial Formula | |
| Y1= (-2290924.07503319*((X/1000)$^4$))+(9585130.51574397*((X/1000)$^3$))-(15034840.326165*((X/1000)$^2$))+(10478707.5382912*(X/1000))-(2738073.65283998) | [1000-1004] |
| Linear Brix Formula | |
| Y2= ((X/1000)*212.43)-210.34 | [1005-1019] |
| Brix Polynomial Formula | |
| Y1= (-2290924.07503319*((X/1000)$^4$))+(9585130.51574397*((X/1000)$^3$))-(15034840.326165*((X/1000)$^2$))+(10478707.5382912*(X/1000))-(2738073.65283998) | [1020-1084] |
| Polynomial Formula | |
| Y3= ((-2084271.57477598*((X/1000)$^4$))+(10138258.1876308*((X/1000)$^3$))-(18520359.784885*((X/1000)$^2$))+(15258063.7784361 *(X/1000))-(4792906.52426027))/1000 | [1085-1130] |

where X corresponds to the density of the must.

The Brix Polynomial formula in the density range [1000-1004] presents slightly curved (nonlinear) values, so a Y1 polynomial equation is used.

The Brix values correlated for densities in the range [1020-1084] and up to [1130] behave in the same manner than in [1000-1004] density ranges with "curved" values. For this reason, two Y1 and Y3 polynomial formulas are used with $R^2$=1 and only in the range where the curve isn't directly proportional. It is worth mentioning that in regard to these polynomial equations two of them were used because of for Brix values >20 the curve of correlated Brix values is longer, so it is recalculated using another polynomial equation with $R^2$=1; this is applicable for the [1020-1130] density rang, different to the linear formula expressed in the [1005 to 1019] range where the density values exhibit a proportional behavior to the correlated Brix values.

If all four musts have values related to each other, the polynomial and linear formulas used are the same.

To calculate the real alcohol volume percentage the following formula can be used:

$$\text{Real Alcohol Volume} = \frac{(\text{Higher Brix} - \text{Lower Brix})}{1.6}$$

The percentage of theoretical alcohol volume can be calculated as:

$$\text{Theoretical Alcohol Volume} = \left[\frac{(HB X 0.83)(55)}{0.855489}\right] \div 100$$

where HB is the Higher Brix.

Finally, the fermentation efficiency can be calculated as:

$$\text{Fermentation Efficiency (\%)} = \frac{\text{Real Alcohol Volume}}{\text{Theoretical Alcohol Volume}} X 100.$$

In some embodiments, the invention also computes the total reducing sugars, which can be calculated as:

$$\text{Total Reducing Sugars} = (^{\circ}Brix)\left(0.83 \frac{\%Total\ Reducing\ Sugars}{^{\circ}Brix}\right)$$

where $\underline{o}$Brix is the Degrees Brix.

The pH and the temperature data enable the system to be more robust and accurate since a larger number of variables can be considered for the fermentation process.

Present invention thus allows the monitoring of the required variables in real time and its automation, as it is possible to program control algorithms that manipulate the process and issue alarms when the data takes unwanted values.

The software used in the present invention, which is related to the programming of the PLC (6), implements the algorithms (i.e. the set of ordered operations) that allow the calculation of the above-mentioned parameters (based on the data acquired/measured by the different sensors (2-5) installed/included in the fermentation tank (1). Particularly, the algorithms are formulas which were developed by the authors of the present invention.

The issued alerts to warn the user when any monitored or computer parameter is out of the permissible range are another important part of the present invention as the allow an operator to make decisions on time to act during the process and so avoid mistakes.

Likewise, the register of the computed information in the cloud enables the comparison between different fermentation processes.

Following different examples are detailed.

**Examples:**

**Example 1: Comparative among other systems with measurement sensors**

The main differences between the proposed monitoring system and a known winemaking system are summarized in the following table 1. Some of the main advantages of the proposed system are that it can measure the parameters in murky or dark musts and the measure considers the entire volume contained in the fermentation tank (1) and not just a fraction like other systems (with the hydrometer).

**Table 1**

| Integrated measuring system for fermentation monitoring: | Application of sensors for control on winemaking line: |
|---|---|
| Static measurement mechanics. | Dynamics measurement mechanics. |
| The measurements of the whole fluid are made in real time. | The measurements are made over a small part of the flow passing through the Coriolis sensor. |
| The system is developed for any type of alcoholic fermentation. | The system is implemented in red wine fermentations. |
| The variables to attain are: Pressure, level, temperature, pH, density, Degrees Brix, alcohol volume percentage, and fermentation efficiency. | Main systems that are intended to cover: pump-over system, measuring system and refrigeration system. |
| Data monitoring is performed at a sampling speed of 8ms. | The results are displayed in a computer. |
| The information is displayed in an HMI screen locally and through different devices remotely. | -------- |
| The entire measuring system is automated making a data record in a determined time and creating statistics of the monitored samplings. It shows simultaneously the numeric value and graphics of the desired variables in real time. | The selection of the functions is generated manually. The automatic operation results in a percentage of the volume in the deposit every certain time. |
| Temperature control. | Temperature control. |

**Example 2: Alcoholic fermentation monitoring system in real time for agave juice must.**

For the production of the alcoholic beverage known as tequila, agave juice has to be fermented. The process lasts for 24-36 hours approximately. Nevertheless, tt should be noted that such time depends on the way every tequila manufacturer makes its own fermentation process since sometimes can be longer.

That is how the proposed monitoring system can be adapted to every type of fermentation process. While performing these tests the processes were monitored and sampled with the hydrometer every two hours like in a real process, recording the data of every sample and comparing the data displayed at the same time by the Brix measurement monitoring system in an alcoholic fermentation.

In this second example, two tests were made. The data obtained is shown below, with a one-hour margin in test 1 and a two-hour margin in test 2. The results can be observed in tables 2 and 3.

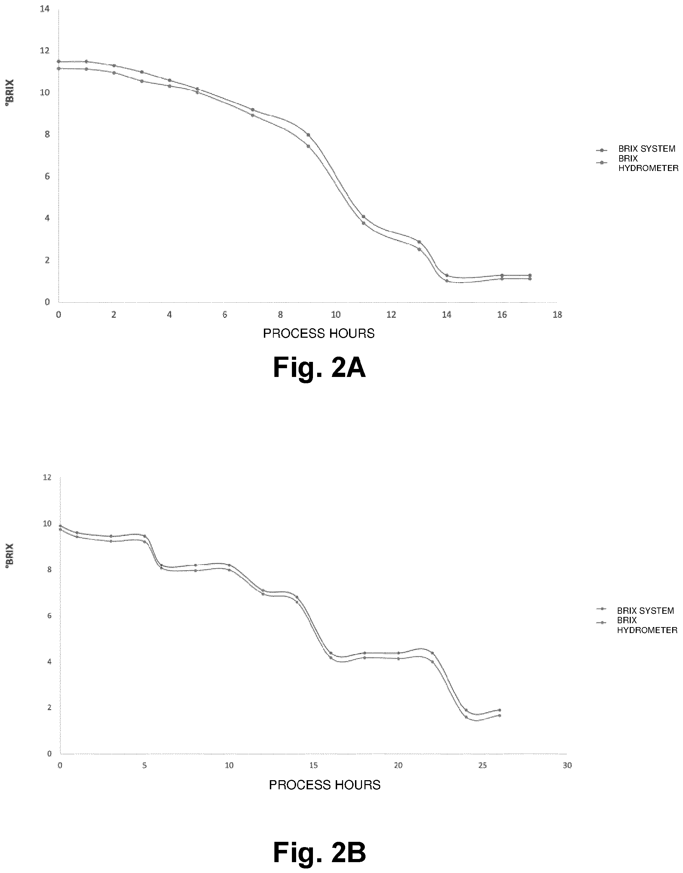

Figs. 2A and 2B show the comparison of the results of the proposed Brix system measurement by means of PLC vs. hydrometer. The information recorded in the graphics shows the behavior of the Brix measurement, regarding the fermentation time for the must with agave juice. It can be seen that the measurement of the Degrees Brix with the proposed system matches with the values measured by the hydrometer; thus, it can be concluded that the proposed system measures efficiently and correctly the Degrees Bris, being those inside the permissible error margin.

# T a b l e  2

| | | | | | | | | | | | | | | | |
|---|---|---|---|---|---|---|---|---|---|---|---|---|---|---|---|
| | | | | | | TEST 1; MEDIA: AGAVE JUICE | | | | | | | | | |
| | Date | Hour | Height Vega Sensor (m) | Pressure Vega Sensor (Pa) | Density by system (sensors) | Brix calculated by system (°Bx) | Brix by Hydrometer (°Bx) | | % DIFFERENCE | System Temperature (°C) | Temperature T. Pattern (°C) | % DIFFERENCE | pH System | pH S. Pattern | % DIFFERENCE |
| 1 | 01/02/22 | 15:14 | 0.598 | 6205 | 1048 | 11.50 | Section 1 | 11.16 | 2.94% | 28.5 | 30.27 | -6.20% | 4.5 | 4.5 | 0.00% |
| | | | | | | | Section 2 | 11.16 | 2.94% | | | | | | |
| | | | | | | | Section 3 | 11.16 | 2.94% | | | | | | |
| | | | | | | | Average | 11.16 | 2.94% | | | | | | |
| 2 | 01/02/22 | 16:32 | 0.598 | 6206 | 1047 | 11.50 | Section 1 | 11.06 | 3.81% | 29.3 | 29.37 | -0.23% | 4.3 | 4.2 | -2.38% |
| | | | | | | | Section 2 | 11.16 | 2.94% | | | | | | |
| | | | | | | | Section 3 | 11.16 | 2.94% | | | | | | |
| | | | | | | | Average | 11.13 | 3.23% | | | | | | |
| 3 | 01/02/22 | 17:20 | 0.598 | 6205 | 1046 | 11.30 | Section 1 | 10.96 | 2.99% | 29.1 | 29.47 | -1.26% | 4.3 | 4.2 | -2.38% |
| | | | | | | | Section 2 | 10.96 | 2.99% | | | | | | |
| | | | | | | | Section 3 | 10.96 | 2.99% | | | | | | |
| | | | | | | | Average | 10.96 | 2.99% | | | | | | |
| 4 | 01/02/22 | 18:20 | 0.598 | 6202 | 1043 | 11 | Section 1 | 10.56 | 3.98% | 29.1 | 29.37 | -0.91% | 4.2 | 4.2 | 0.00% |
| | | | | | | | Section 2 | 10.56 | 3.98% | | | | | | |
| | | | | | | | Section 3 | 10.56 | 3.98% | | | | | | |
| | | | | | | | Average | 10.56 | 3.98% | | | | | | |
| 5 | 01/02/22 | 19:30 | 0.598 | 6188 | 1041 | 10.6 | Section 1 | 10.36 | 2.25% | 30 | 30.57 | -1.89% | 4 | 4.1 | 2.44% |
| | | | | | | | Section 2 | 10.36 | 2.25% | | | | | | |
| | | | | | | | Section 3 | 10.26 | 3.19% | | | | | | |
| | | | | | | | Average | 10.33 | 2.56% | | | | | | |
| 6 | 01/02/22 | 20:30 | 0.598 | 6167 | 1038 | 10.2 | Section 1 | 10.06 | 1.35% | 30 | 30.97 | -3.22% | 4 | 4.1 | 2.44% |
| | | | | | | | Section 2 | 10.06 | 1.35% | | | | | | |
| | | | | | | | Section 3 | 9.96 | 2.33% | | | | | | |
| | | | | | | | Average | 10.03 | 1.68% | | | | | | |
| 7 | 01/02/22 | 22:09 | 0.598 | 6129 | 1032 | 9.2 | Section 1 | 8.89 | 3.34% | 31.3 | 31.97 | -2.13% | 4 | 4 | 0.00% |

| # | Date | Hour | Height Vega Sensor (m) | Pressure Vega Sensor (Pa) | Density by system (sensors) (kg/m3) | Brix calculated by system (°Bx) | Section | Brix by Hydrometer (°Bx) | % DIFFERENCE | System Temperature (°C) | Temperature T. Pattern (°C) | % DIFFERENCE | pH System | pH S. Pattern | % DIFFERENCE |
|---|---|---|---|---|---|---|---|---|---|---|---|---|---|---|---|
| | | | | | | | Section 2 | 8.89 | 3.34% | | | | | | |
| | | | | | | | Section 3 | 8.99 | 2.25% | | | | | | |
| | | | | | | | Average | 8.93 | 2.97% | | | | | | |
| 8 | 02/02/22 | 12:00 | 0.598 | 6093 | 1024 | 8 | Section 1 | 7.29 | 8.84% | 32.5 | 32.97 | -1.43% | 3.9 | 4 | 2.50 |
| | | | | | | | Section 2 | 7.29 | 8.84% | | | | | | |
| | | | | | | | Section 3 | 7.79 | 2.59% | | | | | | |
| | | | | | | | Average | 7.46 | 6.75% | | | | | | |
| 9 | 02/02/22 | 2:30 | 0.598 | 6000 | 1009 | 4.1 | Section 1 | 3.69 | 9.93% | 34.7 | 33.97 | 2.12% | 3.8 | 3.9 | 2.56% |
| | | | | | | | Section 2 | 3.79 | 7.49% | | | | | | |
| | | | | | | | Section 3 | 3.89 | 5.05% | | | | | | |
| | | | | | | | Average | 3.79 | 7.49% | | | | | | |
| 10 | 02/02/22 | 4:30 | 0.598 | 5977 | 1006 | 2.9 | Section 1 | 2.54 | 12.31% | 34.6 | 34.07 | 1.54% | 3.8 | 3.8 | 0.00% |
| | | | | | | | Section 2 | 2.49 | 14.03% | | | | | | |
| | | | | | | | Section 3 | 2.59 | 10.59% | | | | | | |
| | | | | | | | Average | 2.54 | 12.31% | | | | | | |
| 11 | 02/02/22 | 5:55 | 0.597 | 5935 | 1002 | 1.3 | Section 1 | 1.09 | 15.92% | 33.5 | 34.07 | -1.69% | 3.8 | 3.8 | 0.00% |
| | | | | | | | Section 2 | 1.09 | 15.92% | | | | | | |
| | | | | | | | Section 3 | 0.89 | 31.31% | | | | | | |
| | | | | | | | Average | 1.03 | 21.05% | | | | | | |
| 12 | 02/02/22 | 7:50 | 0.595 | 5926 | 1002 | 1.3 | Section 1 | 1.09 | 15.92% | 32.5 | 34.17 | -5.13% | 3.8 | 3.8 | 0.00% |
| | | | | | | | Section 2 | 1.09 | 15.92% | | | | | | |
| | | | | | | | Section 3 | 1.19 | 8.23% | | | | | | |
| | | | | | | | Average | 1.13 | 13.36% | | | | | | |
| 13 | 02/02/22 | 8:40 | 0.597 | 5941 | 1002 | 1.3 | Section 1 | 1.09 | 15.92% | 33.3 | 33.57 | -0.80% | 3.8 | 3.8 | 0.00% |

# Table 3

| # | Date | Hour | Height Vega Sensor (m) | Pressure Vega Sensor (Pa) | Density by system (sensors) (kg/m3) | Brix calculated by system (°Bx) | Section | Brix by Hydrometer (°Bx) | % DIFFERENCE | System Temperature (°C) | Temperature T. Pattern (°C) | % DIFFERENCE | pH System | pH S. Pattern | % DIFFERENCE |
|---|---|---|---|---|---|---|---|---|---|---|---|---|---|---|---|
| | | | | | | | TEST 2; MEDIA: AGAVE JUICE | | | | | | | | |
| 1 | 15-03-2022 | 02:00 pm | 0.645 | 6650 | 1040 | 9.9 | Section 1 | 9.73 | 1.69% | 33 | 33.47 | -1.41% | 4.5 | 4.5 | 0.00% |
| | | | | | | | Section 2 | 9.73 | 1.69% | | | | | | |
| | | | | | | | Section 3 | 9.72 | 1.79% | | | | | | |
| | | | | | | | Average | 9.73 | 1.72% | | | | | | |
| 2 | 15-03-2022 | 03:45 pm | 0.645 | 6635 | 1038 | 9.6 | Section 1 | 9.43 | 1.74% | 33 | 32.47 | 1.62% | 4.5 | 4.4 | -2-27% |
| | | | | | | | Section 2 | 9.43 | 1.74% | | | | | | |
| | | | | | | | Section 3 | 9.39 | 2.16% | | | | | | |
| | | | | | | | Average | 9.42 | 1.88% | | | | | | |
| 3 | 15-03-2022 | 05:45 pm | 0.645 | 6617 | 1037 | 9.45 | Section 1 | 9.19 | 2.72% | 34 | 32.97 | 3.04% | 4.4 | 4.4 | 0.00% |
| | | | | | | | Section 2 | 9.19 | 2.72% | | | | | | |
| | | | | | | | Section 3 | 9.27 | 1.87% | | | | | | |
| | | | | | | | Average | 9.22 | 2.44% | | | | | | |
| 4 | 15-03-2022 | 07:12 pm | 0.645 | 6601 | 1035 | 9.45 | Section 1 | 9.19 | 2.72% | 34 | 33.47 | 1.57% | 4.4 | 4.3 | -2.33% |
| | | | | | | | Section 2 | 9.19 | 2.72% | | | | | | |
| | | | | | | | Section 3 | 9.21 | 2.51% | | | | | | |
| | | | | | | | Average | 9.20 | 2.65% | | | | | | |
| 5 | 15-03-2022 | 08:15 pm | 0.645 | 6587 | 1030 | 8.2 | Section 1 | 7.99 | 2.52% | 35 | 33.97 | 2.95% | 4.3 | 4.2 | -2.38% |
| | | | | | | | Section 2 | 8.09 | 1.30% | | | | | | |
| | | | | | | | Section 3 | 8.09 | 1.30% | | | | | | |

| | | | | | | | | | | | | | | | |
|---|---|---|---|---|---|---|---|---|---|---|---|---|---|---|---|
| | | | | | | | Average | 8.06 | 1.71% | | | | | | |
| 6 | 15-03-2022 | 10:20 pm | 0.642 | 6554 | 1029 | 8.2 | Section 1 | 7.89 | 3.74% | 35 | 33.97 | 2.95% | 4.2 | 4.2 | 0.00% |
| | | | | | | | Section 2 | 7.89 | 3.74% | | | | | | |
| | | | | | | | Section 3 | 8.09 | 1.30% | | | | | | |
| | | | | | | | Average | 7.96 | 2.93% | | | | | | |
| 7 | 16-03-2022 | 12:00 am | 0.642 | 6528 | 1025 | 8.2 | Section 1 | 7.89 | 3.74% | 35 | 33.97 | 2.95% | 4.1 | 4.1 | 0.00% |
| | | | | | | | Section 2 | 7.99 | 2.52% | | | | | | |
| | | | | | | | Section 3 | 8.04 | 1.91% | | | | | | |
| | | | | | | | Average | 7.89 | 2.73% | | | | | | |
| 8 | 16-03-2022 | 02:03 am | 0.642 | 6512 | 1022 | 7.1 | Section 1 | 6.79 | 4.32% | 36 | 34.97 | 2.87% | 4.1 | 4 | -2.50% |
| | | | | | | | Section 2 | 7.04 | 0.80% | | | | | | |
| | | | | | | | Section 3 | 6.99 | 1.51% | | | | | | |
| | | | | | | | Average | 6.94 | 2.21% | | | | | | |
| 9 | 16-03-2022 | 04:20 am | 0.642 | 6495 | 1019 | 6.8 | Section 1 | 6.58 | 3.19% | 36 | 33.97 | 5.65% | 4.1 | 4.1 | 0.00% |
| | | | | | | | Section 2 | 6.49 | 4.51% | | | | | | |
| | | | | | | | Section 3 | 6.69 | 1.57% | | | | | | |
| | | | | | | | Average | 6.59 | 3.09% | | | | | | |
| 10 | 16-03-2022 | 06:04 am | 0.642 | 6476 | 1016 | 4.38 | Section 1 | 4.09 | 6.55% | 36 | 33.97 | 5.65% | 4 | 4 | 0.00% |
| | | | | | | | Section 2 | 4.09 | 6.55% | | | | | | |
| | | | | | | | Section 3 | 4.29 | 1.99% | | | | | | |
| | | | | | | | Average | 4.16 | 5.03% | | | | | | |
| 11 | 16-03-2022 | 08:01 am | 0.642 | 6458 | 1012 | 4.38 | Section 1 | 4.09 | 6.55% | 35 | 33.97 | 2.95% | 4 | 3.9 | -2.56% |
| | | | | | | | Section 2 | 4.19 | 4.27% | | | | | | |
| | | | | | | | Section 3 | 4.19 | 4.27% | | | | | | |
| | | | | | | | Average | 4.16 | 5.03% | | | | | | |
| 12 | 16-03-2022 | 10:20 am | 0.638 | 6437 | 1015 | 4.38 | Section 1 | 4.09 | 6.55% | 35 | 33.97 | 2.95% | 4 | 3.9 | -2.56% |
| | | | | | | | Section 2 | 4.09 | 6.55% | | | | | | |
| | | | | | | | Section 3 | 4.19 | 4.27% | | | | | | |
| | | | | | | | Average | 4.13 | 5.79% | | | | | | |
| 13 | 16-03-2022 | 12:00 pm | 0.638 | 6428 | 1014 | 4.38 | Section 1 | 3.793 | 13.40% | 35 | 33.97 | 2.95% | 3.9 | 3.9 | 0.00% |
| | | | | | | | Section 2 | 3.993 | 8.84% | | | | | | |
| | | | | | | | Section 3 | 4.193 | 4.27% | | | | | | |
| | | | | | | | Average | 3.99 | 8.84% | | | | | | |
| 14 | 16-03-2022 | 02:43 pm | 0.638 | 6423 | 1007 | 1.89 | Section 1 | 1.29 | 31.59% | 36 | 34.97 | 2.87% | 3.8 | 3.8 | 0.00% |
| | | | | | | | Section 2 | 1.69 | 10.42% | | | | | | |
| | | | | | | | Section 3 | 1.79 | 5.13% | | | | | | |
| | | | | | | | Average | 1.59 | 15.71% | | | | | | |
| 15 | 16-03-2022 | 04:25 pm | 0.638 | 6421 | 1006 | 1.89 | Section 1 | 1.593 | 15.71% | 35 | 33.97 | 2.95% | 3.8 | 3.8 | 0.00% |
| | | | | | | | Section 2 | 1.593 | 15.71% | | | | | | |
| | | | | | | | Section 3 | 1.792 | 5.13% | | | | | | |
| | | | | | | | Average | 1.66 | 12.19% | | | | | | |

The results obtained from the previous tests were satisfactory because:

1) The records obtained by the proposed system remained within the tolerance and error margin of 15%.
2) The measurements were taken using measuring instruments for Brix, temperature, and pH, traceable to international standards (i.e. the primary measuring instruments were calibrated by international standards from the National Metrology Center).
3) The aforementioned results are reliable measurements from every monitored variable for agave juice.

**Example 3: Alcoholic fermentation monitoring system in real time from barely wort/must**

For the production of beer it is necessary to ferment the wort through the cooking process of the malt. The beer fermentation process takes around 10 days. While making these tests it was extremely important to monitor and take samples every two hours with the hydrometer like in a real process. The data from each sample was also recorded and compared with the data obtained by the proposed monitoring system. Two tests were made, and the results can be observed in tables 4 and 5 below.

## Table 4

| | Date | Hour | Height Vega Sensor (m) | Pressure Vega Sensor (Pa) | Density by system (sensors) (kg/m3) | Brix calculated by system (°Bx) | Brix by Hydrometer (°Bx) | | % DIFFERENCE | System Temperature (°C) | Temperature T. Pattern (°C) | % DIFFERENCE | pH System | pH S. Pattern | % DIFFERENCE |
|---|---|---|---|---|---|---|---|---|---|---|---|---|---|---|---|
| 1 | 13/06/22 | 11:00 PM | NOT APPLICABLE | NOT APPLICABLE | NOT APPLICABLE | 14.58 | SECTION 1 | 14.47 | 0.74% | 28.8 | 28.47 | 1.16% | 4.6 | 4.6 | 0.00% |
| | | | | | | | SECTION 2 | 14.47 | 0.74% | | | | | | |
| | | | | | | | SECTION 3 | 14.47 | 0.74% | | | | | | |
| | | | | | | | AVERAGE | 14.47 | 0.74% | | | | | | |
| 2 | 14/06/22 | 08:00 AM | NOT APPLICABLE | NOT APPLICABLE | NOT APPLICABLE | 14.4 | SECTION 1 | 14.35 | 0.33% | 26.5 | 26.97 | -1.76% | 4.6 | 4.6 | 0.00% |
| | | | | | | | SECTION 2 | 14.35 | 0.33% | | | | | | |
| | | | | | | | SECTION 3 | 14.35 | 0.33% | | | | | | |
| | | | | | | | AVERAGE | 14.35 | 0.33% | | | | | | |
| 3 | 14/06/22 | 10:00 AM | NOT APPLICABLE | NOT APPLICABLE | NOT APPLICABLE | 14.3 | SECTION 1 | 14.36 | -0.43% | 27 | 26.766 | 0.87% | 4.6 | 4.6 | 0.00% |
| | | | | | | | SECTION 2 | 14.36 | -0.43% | | | | | | |
| | | | | | | | SECTION 3 | 14.36 | -0.43% | | | | | | |
| | | | | | | | AVERAGE | 14.36 | -0.43% | | | | | | |
| 4 | 14/06/22 | 12:00 AM | NOT APPLICABLE | NOT APPLICABLE | NOT APPLICABLE | 14 | SECTION 1 | 13.96 | 0.27% | 28.5 | 27.97 | 1.87% | 4.6 | 4.5 | -2.22% |
| | | | | | | | SECTION 2 | 13.96 | 0.27% | | | | | | |
| | | | | | | | SECTION 3 | 13.96 | 0.27% | | | | | | |
| | | | | | | | AVERAGE | 13.96 | 0.27% | | | | | | |
| 5 | 14/06/22 | 02:00 PM | NOT APPLICABLE | NOT APPLICABLE | NOT APPLICABLE | 13.5 | SECTION 1 | 13.46 | 0.28% | 28 | 27.47 | 1.91% | 4.6 | 4.5 | -2-22% |
| | | | | | | | SECTION 2 | 13.46 | 0.28% | | | | | | |
| | | | | | | | SECTION 3 | 13.46 | 0.28% | | | | | | |
| | | | | | | | AVERAGE | 13.46 | 0.28% | | | | | | |
| 6 | 14/06/22 | 04:00 PM | NOT APPLICABLE | NOT APPLICABLE | NOT APPLICABLE | 12.3 | SECTION 1 | 12.76 | -3.76% | 28.4 | 28.97 | -1.99% | 4.5 | 4.5 | 0.00% |
| | | | | | | | SECTION 2 | 12.76 | -3.76% | | | | | | |
| | | | | | | | SECTION 3 | 12.76 | -3.76% | | | | | | |
| | | | | | | | AVERAGE | 12.76 | -3.76% | | | | | | |
| 7 | 14/06/22 | 06:00 PM | NOT APPLICABLE | NOT APPLICABLE | NOT APPLICABLE | 11.6 | SECTION 1 | 12.36 | -6.57% | 29 | 29.47 | -1.61% | 4.5 | 4.5 | 0.00% |
| | | | | | | | SECTION 2 | 12.36 | -6.57% | | | | | | |

TEST 1; MEDIA: MALT

| # | Date | Time | | | | | Section | | | | | | | | |
|---|---|---|---|---|---|---|---|---|---|---|---|---|---|---|---|
| | | | | | | | SECTION 3 | 12.36 | -6.57% | | | | | | |
| | | | | | | | AVERAGE | 12.36 | -6.57% | | | | | | |
| 8 | 14/06/22 | 08:00 PM | NOT APPLICABLE | NOT APPLICABLE | NOT APPLICABLE | 11.4 | SECTION 1 | 10.36 | 9.11% | 29 | 28.47 | 1.84% | 4.5 | 4.5 | 0.00% |
| | | | | | | | SECTION 2 | 10.36 | 9.11% | | | | | | |
| | | | | | | | SECTION 3 | 10.36 | 9.11% | | | | | | |
| | | | | | | | AVERAGE | 10.36 | 9.11% | | | | | | |
| 9 | 15/06/22 | 08:00 AM | NOT APPLICABLE | NOT APPLICABLE | NOT APPLICABLE | 10.9 | SECTION 1 | 11.26 | -3.32% | 27.5 | 27.0 | 1.94% | 4.5 | 4.4 | -2.27% |
| | | | | | | | SECTION 2 | 11.26 | -3.32% | | | | | | |
| | | | | | | | SECTION 3 | 11.26 | -3.32% | | | | | | |
| | | | | | | | AVERAGE | 11.26 | -3.32% | | | | | | |
| 10 | 15/06/22 | 10:00 AM | NOT APPLICABLE | NOT APPLICABLE | NOT APPLICABLE | 10.8 | SECTION 1 | 10.86 | -0.57% | 27.4 | 26.97 | 1.58% | 4.4 | 4.4 | 0.00% |
| | | | | | | | SECTION 2 | 10.86 | -0.57% | | | | | | |
| | | | | | | | SECTION 3 | 10.86 | -0.57% | | | | | | |
| | | | | | | | AVERAGE | 10.86 | -0.57% | | | | | | |
| 11 | 15/06/22 | 12:00 PM | NOT APPLICABLE | NOT APPLICABLE | NOT APPLICABLE | 11.03 | SECTION 1 | 10.76 | 2.43% | 28 | 27.47 | 1.91% | 4.4 | 4.4 | 0.00% |
| | | | | | | | SECTION 2 | 10.76 | 2.43% | | | | | | |
| | | | | | | | SECTION 3 | 10.76 | 2.43% | | | | | | |
| | | | | | | | AVERAGE | 10.76 | 2.43% | | | | | | |
| 12 | 15/06/22 | 02:00 PM | NOT APPLICABLE | NOT APPLICABLE | NOT APPLICABLE | 10.6 | SECTION 1 | 10.50 | 0.92% | 28.9 | 28.47 | 1.50% | 4.4 | 4.3 | -2.33% |
| | | | | | | | SECTION 2 | 10.50 | 0.92% | | | | | | |
| | | | | | | | SECTION 3 | 10.50 | 0.92% | | | | | | |
| | | | | | | | AVERAGE | 10.50 | 0.92% | | | | | | |
| 13 | 15/06/22 | 04:00 PM | NOT APPLICABLE | NOT APPLICABLE | NOT APPLICABLE | 10.2 | SECTION 1 | 10.16 | 0.37% | 29.3 | 28.77 | 1.82% | 4.3 | 4.3 | 0.00% |
| | | | | | | | SECTION 2 | 10.16 | 0.37% | | | | | | |
| | | | | | | | SECTION 3 | 10.16 | 0.37% | | | | | | |
| | | | | | | | AVERAGE | 10.16 | 0.37% | | | | | | |
| 14 | 15/06/22 | 06:00 PM | NOT APPLICABLE | NOT APPLICABLE | NOT APPLICABLE | 9.39 | SECTION 1 | 9.69 | -3.23% | 29.5 | 28.97 | 1.81% | 4.3 | 4.3 | 0.00% |
| | | | | | | | SECTION 2 | 9.69 | -3.23% | | | | | | |
| | | | | | | | SECTION 3 | 9.69 | -3.23% | | | | | | |
| | | | | | | | AVERAGE | 9.69 | -3.23% | | | | | | |
| 15 | 15/06/22 | 08:00 PM | NOT APPLICABLE | NOT APPLICABLE | NOT APPLICABLE | 8.2 | SECTION 1 | 8.19 | 0.09% | 29 | 28.67 | 1.15% | 4.3 | 4.3 | 0.00% |
| | | | | | | | SECTION 2 | 8.19 | 0.09% | | | | | | |
| | | | | | | | SECTION 3 | 8.19 | 0.09% | | | | | | |
| | | | | | | | AVERAGE | 8.19 | 0.09% | | | | | | |
| 16 | 16/06/22 | 08:00 AM | NOT APPLICABLE | NOT APPLICABLE | NOT APPLICABLE | 6.5 | SECTION 1 | 6.29 | 3.18% | 29.8 | 29.67 | 0.45% | 4.3 | 4.3 | 0.00% |
| | | | | | | | SECTION 2 | 6.29 | 3.18% | | | | | | |
| | | | | | | | SECTION 3 | 6.29 | 3.18% | | | | | | |
| | | | | | | | AVERAGE | 6.29 | 3.18% | | | | | | |
| 17 | 16/06/22 | 10:00 AM | NOT APPLICABLE | NOT APPLICABLE | NOT APPLICABLE | 6 | SECTION 1 | 6.0 | 0.12% | 29 | 28.77 | 0.81% | 4.2 | 4.2 | 0.00% |
| | | | | | | | SECTION 2 | 6.0 | 0.12% | | | | | | |
| | | | | | | | SECTION 3 | 6.0 | 0.12% | | | | | | |
| | | | | | | | AVERAGE | 5.99 | 0.12% | | | | | | |
| 18 | 16/06/22 | 12:00 PM | NOT APPLICABLE | NOT APPLICABLE | NOT APPLICABLE | 5.6 | SECTION 1 | 5.5 | 1.91% | 29.5 | 29.27 | 0.79% | 4.2 | 4.2 | 0.00% |
| | | | | | | | SECTION 2 | 5.5 | 1.91% | | | | | | |
| | | | | | | | SECTION 3 | 5.5 | 1.91% | | | | | | |
| | | | | | | | AVERAGE | 5.49 | 1.91% | | | | | | |
| 19 | 16/06/22 | 02:00 PM | NOT APPLICABLE | NOT APPLICABLE | NOT APPLICABLE | 5.4 | SECTION 1 | 5.5 | -1.72% | 30.3 | 29.97 | 1.10% | 4.2 | 4.2 | 0.00% |
| | | | | | | | SECTION 2 | 5.5 | -1.72% | | | | | | |
| | | | | | | | SECTION 3 | 5.5 | -1.72% | | | | | | |
| | | | | | | | AVERAGE | 5.49 | -1.72% | | | | | | |
| 20 | 16/06/22 | 04:00 PM | NOT APPLICABLE | NOT APPLICABLE | NOT APPLICABLE | 5.3 | SECTION 1 | 5.3 | 0.13% | 30 | 29.27 | 2.45%% | 4.2 | 4.1 | -2.44% |
| | | | | | | | SECTION 2 | 5.3 | 0.13% | | | | | | |
| | | | | | | | SECTION 3 | 5.3 | 0.13% | | | | | | |

| # | Date | Hour | Height Vega Sensor (m) | Pressure Vega Sensor (Pa) | Density by system (kg/m3) | Brix calculated by system (°Bx) | Brix by Hydrometer (°Bx) | | % DIFFERENCE | System Temperature (°C) | Temperature T. Pattern (°C) | % DIFFERENCE | pH System | pH S. Pattern | % DIFFERENCE |
|---|---|---|---|---|---|---|---|---|---|---|---|---|---|---|---|
| | | | | | | | AVERAGE | 5.29 | 0.13% | | | | | | |
| 21 | 16/06/22 | 06:00 PM | NOT APPLICABLE | NOT APPLICABLE | NOT APPLICABLE | 4.7 | SECTION 1 | 4.9 | -4.11% | 28.9 | 28.47 | 1.50% | 4.1 | 4.1 | 0.00% |
| | | | | | | | SECTION 2 | 4.9 | -4.11% | | | | | | |
| | | | | | | | SECTION 3 | 4.9 | -4.11% | | | | | | |
| | | | | | | | AVERAGE | 4.89 | -4.11% | | | | | | |
| 22 | 16/06/22 | 08:00 PM | NOT APPLICABLE | NOT APPLICABLE | NOT APPLICABLE | 4.5 | SECTION 1 | 4.5 | 0.16% | 28.5 | 27.97 | 1.87% | 4.1 | 4.1 | 0.00% |
| | | | | | | | SECTION 2 | 4.5 | 0.16% | | | | | | |
| | | | | | | | SECTION 3 | 4.5 | 0.16% | | | | | | |
| | | | | | | | AVERAGE | 4.49 | 0.16% | | | | | | |
| 23 | 17/06/22 | 08:00 AM | NOT APPLICABLE | NOT APPLICABLE | NOT APPLICABLE | 4.8 | SECTION 1 | 4.5 | 6.40% | 26.4 | 25.97 | 1.64% | 4.1 | 4 | -2.50% |
| | | | | | | | SECTION 2 | 4.5 | 6.40% | | | | | | |
| | | | | | | | SECTION 3 | 4.5 | 6.40% | | | | | | |
| | | | | | | | AVERAGE | 4.49 | 6.40% | | | | | | |

# T a b l e   5

| TEST 2; MEDIA: MALT | | | | | | | | | | | | | | | |
|---|---|---|---|---|---|---|---|---|---|---|---|---|---|---|---|
| # | Date | Hour | Height Vega Sensor (m) | Pressure Vega Sensor (Pa) | Density by system (sensors) (kg/m3) | Brix calculated by system (°Bx) | Brix by Hydrometer (°Bx) | | % DIFFERENCE | System Temperature (°C) | Temperature T. Pattern (°C) | % DIFFERENCE | pH System | pH S. Pattern | % DIFFERENCE |
| 1 | 05/07/22 | 11:06 AM | NOT APPLICABLE | NOT APPLICABLE | NOT APPLICABLE | 17.6 | SECTION 1 | 16.86 | 4.19% | 29.8 | 29.97 | -0.56% | 4.6 | 4.6 | 0.00% |
| | | | | | | | SECTION 2 | 16.86 | 4.19% | | | | | | |
| | | | | | | | SECTION 3 | 16.86 | 4.19% | | | | | | |
| | | | | | | | AVERAGE | 16.86 | 4.19% | | | | | | |
| 2 | 05/07/22 | 12:22 PM | NOT APPLICABLE | NOT APPLICABLE | NOT APPLICABLE | 15.2 | SECTION 1 | 14.96 | 1.57% | 30.9 | 30.97 | -0.21% | 4.6 | 4.6 | 0.00% |
| | | | | | | | SECTION 2 | 14.96 | 1.57% | | | | | | |
| | | | | | | | SECTION 3 | 14.96 | 1.57% | | | | | | |
| | | | | | | | AVERAGE | 14.96 | 1.57% | | | | | | |
| 3 | 05/07/22 | 2:40 PM | NOT APPLICABLE | NOT APPLICABLE | NOT APPLICABLE | 14.7 | SECTION 1 | 14.93 | -1.58% | 31 | 30.97 | 0.11% | 4.6 | 4.6 | 0.00% |
| | | | | | | | SECTION 2 | 14.93 | -1.58% | | | | | | |
| | | | | | | | SECTION 3 | 14.93 | -1.58% | | | | | | |
| | | | | | | | AVERAGE | 14.93 | -1.58% | | | | | | |
| 4 | 05/07/22 | 4:50 PM | NOT APPLICABLE | NOT APPLICABLE | NOT APPLICABLE | 14.8 | SECTION 1 | 14.73 | 0.46% | 30.8 | 30.97 | -0.54% | 4.6 | 4.5 | 2.17% |
| | | | | | | | SECTION 2 | 14.73 | 0.46% | | | | | | |
| | | | | | | | SECTION 3 | 14.73 | 0.46% | | | | | | |
| | | | | | | | AVERAGE | 14.73 | 0.46% | | | | | | |
| 5 | 05/07/22 | 6.30 PM | NOT APPLICABLE | NOT APPLICABLE | NOT APPLICABLE | 14.3 | SECTION 1 | 14.36 | -0.43% | 26.9 | 26.97 | -0.25% | 4.5 | 4.5 | 0.00% |
| | | | | | | | SECTION 2 | 14.36 | -0.43% | | | | | | |
| | | | | | | | SECTION 3 | 14.36 | -0.43% | | | | | | |
| | | | | | | | AVERAGE | 14.36 | -0.43% | | | | | | |
| 6 | 05/07/22 | 7:40 PM | NOT APPLICABLE | NOT APPLICABLE | NOT APPLICABLE | 14.3 | SECTION 1 | 14.23 | 0.48% | 26.9 | 26.97 | -0.25% | 4.5 | 4.5 | 0.00% |
| | | | | | | | SECTION 2 | 14.23 | 0.48% | | | | | | |
| | | | | | | | SECTION 3 | 14.23 | 0.48% | | | | | | |
| | | | | | | | AVERAGE | 14.23 | 0.48% | | | | | | |
| 7 | 06/07/22 | 8:00 AM | NOT APPLICABLE | NOT APPLICABLE | NOT APPLICABLE | 15 | SECTION 1 | 14.66 | 2.25% | 23 | 22.97 | 0.15% | 4.5 | 4.5 | 0.00% |
| | | | | | | | SECTION 2 | 14.66 | 2.25% | | | | | | |
| | | | | | | | SECTION 3 | 14.66 | 2.25% | | | | | | |
| | | | | | | | AVERAGE | 14.66 | 2.25% | | | | | | |
| 8 | 06/07/22 | 10:05 AM | NOT APPLICABLE | NOT APPLICABLE | NOT APPLICABLE | 14.8 | SECTION 1 | 14.15 | 4.38% | 23.8 | 23.97 | -0.70% | 4.5 | 4.5 | 0.00% |
| | | | | | | | SECTION 2 | 14.15 | 4.38% | | | | | | |
| | | | | | | | SECTION 3 | 14.15 | 4.38% | | | | | | |
| | | | | | | | AVERAGE | 14.15 | 4.38% | | | | | | |

| | | | | | | | | | | | | | | | |
|---|---|---|---|---|---|---|---|---|---|---|---|---|---|---|---|
| 9 | 06/07/22 | 12:10 PM | NOT APPLICABLE | NOT APPLICABLE | NOT APPLICABLE | 14.8 | SECTION 1 | 14.06 | 4.99% | 23.9 | 23.97 | -0.28% | 4.4 | 4.4 | 0.00% |
| | | | | | | | SECTION 2 | 14.06 | 4.99% | | | | | | |
| | | | | | | | SECTION 3 | 14.06 | 4.99% | | | | | | |
| | | | | | | | AVERAGE | 14.06 | 4.99% | | | | | | |
| 10 | 06/07/22 | 02:20 PM | NOT APPLICABLE | NOT APPLICABLE | NOT APPLICABLE | 14.3 | SECTION 1 | 14.06 | 1.66% | 26 | 25.97 | 0.13% | 4.4 | 4.4 | 0.00% |
| | | | | | | | SECTION 2 | 14.06 | 1.66% | | | | | | |
| | | | | | | | SECTION 3 | 14.06 | 1.66% | | | | | | |
| | | | | | | | AVERAGE | 14.06 | 1.66% | | | | | | |
| 11 | 06/07/22 | 4:27 PM | NOT APPLICABLE | NOT APPLICABLE | NOT APPLICABLE | 14.3 | SECTION 1 | 14.16 | 0.97% | 25.9 | 25.97 | -0.25% | 4.4 | 4.4 | 0.00% |
| | | | | | | | SECTION 2 | 14.16 | 0.97% | | | | | | |
| | | | | | | | SECTION 3 | 14.16 | 0.97% | | | | | | |
| | | | | | | | AVERAGE | 14.16 | 0.97% | | | | | | |
| 12 | 06/07/22 | 6:15 PM | NOT APPLICABLE | NOT APPLICABLE | NOT APPLICABLE | 14.1 | SECTION 1 | 13.96 | 0.98% | 26 | 25.97 | 0.13% | 4.4 | 4.3 | 2.27 |
| | | | | | | | SECTION 2 | 13.96 | 0.98% | | | | | | |
| | | | | | | | SECTION 3 | 13.96 | 0.98% | | | | | | |
| | | | | | | | AVERAGE | 13.96 | 0.98% | | | | | | |
| 13 | 07/07/22 | 8:05 AM | NOT APPLICABLE | NOT APPLICABLE | NOT APPLICABLE | 13.1 | SECTION 1 | 12.22 | 6.70% | 24.3 | 24.47 | -0.68% | 4.3 | 4.3 | 0.00% |
| | | | | | | | SECTION 2 | 12.22 | 6.70% | | | | | | |
| | | | | | | | SECTION 3 | 12.22 | 6.70% | | | | | | |
| | | | | | | | AVERAGE | 12.22 | 6.70% | | | | | | |
| 14 | 07/07/22 | 10:35 AM | NOT APPLICABLE | NOT APPLICABLE | NOT APPLICABLE | 11.6 | SECTION 1 | 11.26 | 2.91% | 26 | 25.97 | 0.13% | 4.3 | 4.3 | 0.00% |
| | | | | | | | SECTION 2 | 11.26 | 2.91% | | | | | | |
| | | | | | | | SECTION 3 | 11.26 | 2.91% | | | | | | |
| | | | | | | | AVERAGE | 11.26 | 2.91% | | | | | | |
| 15 | 08/07/22 | 12:30 PM | NOT APPLICABLE | NOT APPLICABLE | NOT APPLICABLE | 11.2 | SECTION 1 | 10.88 | 2.84% | 27.5 | 27.67 | -0.60% | 4.3 | 4.2 | 2.33% |
| | | | | | | | SECTION 2 | 10.88 | 2.84% | | | | | | |
| | | | | | | | SECTION 3 | 10.88 | 2.84% | | | | | | |
| | | | | | | | AVERAGE | 10.88 | 2.84% | | | | | | |
| 16 | 08/07/22 | 4:08 PM | NOT APPLICABLE | NOT APPLICABLE | NOT APPLICABLE | 10.8 | SECTION 1 | 10.16 | 5.91% | 29.3 | 29.47 | -0.57% | 4.2 | 4.2 | 0.00% |
| | | | | | | | SECTION 2 | 10.16 | 5.91% | | | | | | |
| | | | | | | | SECTION 3 | 10.16 | 5.91% | | | | | | |
| | | | | | | | AVERAGE | 10.16 | 5.91% | | | | | | |
| 17 | 09/07/22 | 8:13 AM | NOT APPLICABLE | NOT APPLICABLE | NOT APPLICABLE | 8.7 | SECTION 1 | 8.69 | 0.08% | 25.3 | 25.47 | -0.66% | 4.2 | 4.2 | 0.00% |
| | | | | | | | SECTION 2 | 8.69 | 0.08% | | | | | | |
| | | | | | | | SECTION 3 | 8.69 | 0.08% | | | | | | |
| | | | | | | | AVERAGE | 8.69 | 0.08% | | | | | | |
| 18 | 10/07/22 | 10:55 AM | NOT APPLICABLE | NOT APPLICABLE | NOT APPLICABLE | 6.8 | SECTION 1 | 6.99 | -2.84% | 28.8 | 28.97 | -0.58% | 4.1 | 4.1 | 0.00% |
| | | | | | | | SECTION 2 | 6.99 | -2.84% | | | | | | |
| | | | | | | | SECTION 3 | 6.99 | -2.84% | | | | | | |
| | | | | | | | AVERAGE | 6.99 | -2.84% | | | | | | |
| 19 | 11/07/22 | 8:10 AM | NOT APPLICABLE | NOT APPLICABLE | NOT APPLICABLE | 6.3 | SECTION 1 | 5.59 | 11.22% | 24 | 23.97 | 0.14% | 4.1 | 4.1 | 0.00% |
| | | | | | | | SECTION 2 | 5.59 | 11.22% | | | | | | |
| | | | | | | | SECTION 3 | 5.59 | 11.22% | | | | | | |
| | | | | | | | AVERAGE | 5.59 | 11.22% | | | | | | |
| | | | | | | | SECTION 2 | | | | | | | | |
| | | | | | | | SECTION 3 | | | | | | | | |
| | | | | | | | AVERAGE | | | | | | | | |

The results obtained from the previous tests were satisfactory because:

1) The records obtained by the proposed system remained within the tolerance and error margin of 15%.
2) The measurements were taken using measuring instruments for Brix, temperature, and pH, traceable to international standards (i.e. the primary measuring instruments were calibrated by international standards from the National Metrology Center).

3) The aforementioned results are reliable measurements from every monitored variable for fermentation of malt.

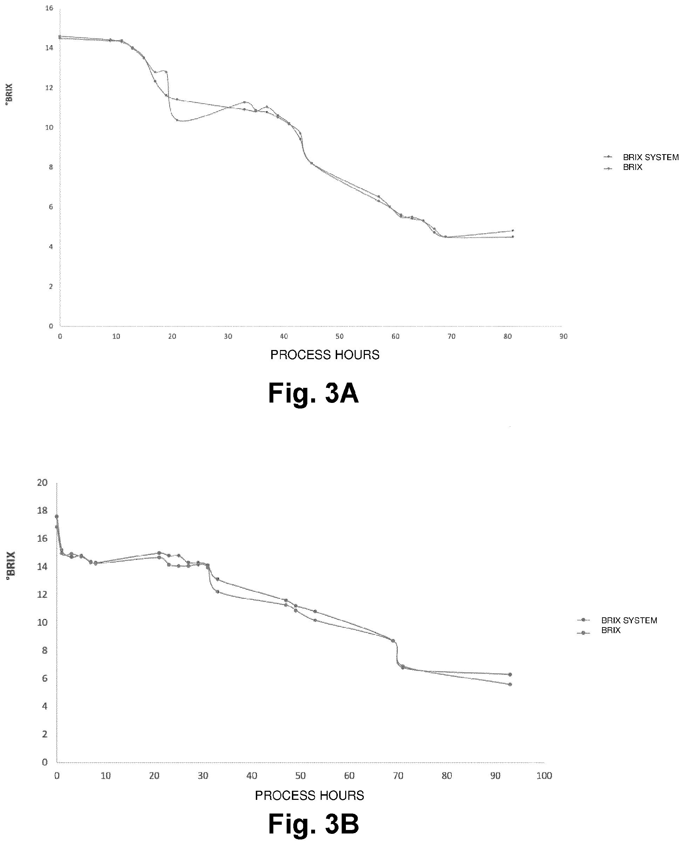

Figs. 3A and 3B show the results of the proposed Brix system by means of PLC vs. hydrometer. The information registered in the graphics shows the behavior of Brix regarding the processing time for the fermentation of malt wort. The Degrees Brix values match in both methods; therefore the proposed monitoring system carries out the measurements within the tolerance and permissible error margin.

**Example 4: Alcoholic fermentation monitoring system in real time for molasses wort/must**

The molasses have to be fermented to elaborate rum (extracted from sugar cane), in a process that lasts 24 hours, although it depends on the type of process. While performing the tests shown in tables 6 and 7 the samples were taken every two hours with the hydrometer and compared with the data shown in the proposed monitoring system.

## Table 6

| | Date | Hour | Height Vega Sensor (m) | Pressure Vega Sensor (Pa) | Density by system (sensors) (kg/m3) | Brix calculated by system (°Bx) | Brix by Hydrometer (°Bx) | | % DIFFERENCE | System Temperature (°C) | Temperature T. Pattern (°C) | % DIFFERENCE | pH System | pH S. Pattern | % DIFFERENCE |
|---|---|---|---|---|---|---|---|---|---|---|---|---|---|---|---|
| | | | | | | | **TEST 1; MEDIA: MOLASSES** | | | | | | | | |
| 1 | 26/01/22 | 9:18 pm | 0.604 | 6356 | 1058 | 13.8 | SECTION 1 | 13.062 | 5% | 25 | 26.17 | -4.66% | 4.4 | 4.2 | -4.76% |
| | | | | | | | SECTION 2 | 13.062 | 5% | | | | | | |
| | | | | | | | SECTION 3 | 12.862 | 7% | | | | | | |
| | | | | | | | AVERAGE | 13.00 | 6% | | | | | | |
| 2 | 27/01/22 | 7:44 am | 0.621 | 6261 | 1026 | 8.5 | SECTION 1 | 8.49 | 0.08% | 28.8 | 28.97 | -0.58% | 4.4 | 4.2 | -4.76% |
| | | | | | | | SECTION 2 | 8.49 | 0.08% | | | | | | |
| | | | | | | | SECTION 3 | 8.19 | 3.61% | | | | | | |
| | | | | | | | AVERAGE | 8.39 | 1.26% | | | | | | |
| 3 | 27/01/22 | 9:00 am | 0.621 | 6231 | 1024 | 8.3 | SECTION 1 | 7.89 | 4.90% | 29.3 | 30.27 | -3.30% | 4.4 | 4.2 | -4.76% |
| | | | | | | | SECTION 2 | 7.89 | 4.90% | | | | | | |
| | | | | | | | SECTION 3 | 7.79 | 6.11% | | | | | | |
| | | | | | | | AVERAGE | 7.86 | 5.31% | | | | | | |
| 4 | 28/01/22 | 11:00 am | 0.614 | 6184 | 1018 | 7 | SECTION 1 | 6.39 | 8.67% | 29.9 | 29.47 | 1.45% | 4.1 | 4.1 | 0.00% |
| | | | | | | | SECTION 2 | 6.39 | 8.67% | | | | | | |
| | | | | | | | SECTION 3 | 6.59 | 5.81% | | | | | | |
| | | | | | | | AVERAGE | 6.46 | 7.72% | | | | | | |
| 5 | 29/01/22 | 12:40 pm | 0.612 | 6173 | 1016 | 6.2 | SECTION 1 | 5.69 | 8.18% | 30.7 | 31.57 | -2.82% | 4.1 | 4 | -2.50% |
| | | | | | | | SECTION 2 | 5.69 | 8.18% | | | | | | |
| | | | | | | | SECTION 3 | 5.79 | 6.56% | | | | | | |
| | | | | | | | AVERAGE | 5.73 | 7.64% | | | | | | |
| 6 | 29/01/22 | 2:20 pm | 0.604 | 6162 | 1015 | 5.9 | SECTION 1 | 5.39 | 8.59% | 30.3 | 31.47 | -3.85% | 4.1 | 4 | -2.50% |
| | | | | | | | SECTION 2 | 5.39 | 8.59% | | | | | | |
| | | | | | | | SECTION 3 | 5.6 | 5.08% | | | | | | |
| | | | | | | | AVERAGE | 5.46 | 7.42% | | | | | | |
| | 29/01/22 | 4:26 pm | 0.605 | 6168 | 1014 | 5.6 | SECTION 1 | 5.29 | 5.48% | 30.6 | 30.77 | -0.54% | 4 | 4 | 0.00% |
| | | | | | | | SECTION 2 | 5.29 | 5.48% | | | | | | |
| | | | | | | | SECTION 3 | 5.39 | 3.70% | | | | | | |
| | | | | | | | AVERAGE | 5.33 | 4.89% | | | | | | |

# Table 7

| No. | Date | Hour | Height Vega Sensor (m) | Pressure Vega Sensor (Pa) | Density by system (sensors) (kg/m3) | Brix calculated by system (°Bx) | Brix by Hydrometer (°Bx) | | % DIFFERENCE | System Temperature (°C) | Temperature T. Pattern (°C) | % DIFFERENCE | pH System | pH S. Pattern | % DIFFERENCE |
|---|---|---|---|---|---|---|---|---|---|---|---|---|---|---|---|
| | | | | | | | TEST 2; MEDIA: MOLASSES | | | | | | | | |
| 1 | 17/02/22 | 2.40 PM | 0.591 | 6254 | 1051 | 15.5 | SECTION 1 | 15.76 | -1.69% | 24.9 | 24.97 | 0.26% | 4.5 | 4.5 | 0.00% |
| | | | | | | | SECTION 2 | 15.76 | -1.69% | | | | | | |
| | | | | | | | SECTION 3 | 15.76 | -1.69% | | | | | | |
| | | | | | | | AVERAGE | 15.76 | -1.69% | | | | | | |
| 2 | 17/02/22 | 6.48 PM | 0.591 | 6261 | 1046 | 15.9 | SECTION 1 | 15.66 | 1.50% | 24.6 | 25.97 | 5.26% | 4.3 | 4.2 | -2.38% |
| | | | | | | | SECTION 2 | 15.66 | 1.50% | | | | | | |
| | | | | | | | SECTION 3 | 15.66 | 1.50% | | | | | | |
| | | | | | | | AVERAGE | 15.66 | 1.50% | | | | | | |
| 3 | 17/02/22 | 8.53 PM | 0.591 | 6244 | 1057.9 | 15.1 | SECTION 1 | 14.86 | 1.58% | 24.8 | 25.97 | 4.49% | 4.3 | 4.2 | -2.38% |
| | | | | | | | SECTION 2 | 14.86 | 1.58% | | | | | | |
| | | | | | | | SECTION 3 | 14.86 | 1.58% | | | | | | |
| | | | | | | | AVERAGE | 14.86 | 1.58% | | | | | | |
| 4 | 17/02/22 | 10:57 PM | 0.591 | 6234 | 1056.9 | 14.5 | SECTION 1 | 14.51 | -0.08% | 26 | 26.77 | 2.86% | 4.2 | 4.2 | 0.00% |
| | | | | | | | SECTION 2 | 14.61 | -0.77% | | | | | | |
| | | | | | | | SECTION 3 | 14.61 | 0.77% | | | | | | |
| | | | | | | | AVERAGE | 14.58 | -0.54% | | | | | | |
| 5 | 18/02/22 | 1:06 AM | 0.591 | 6191 | 1049 | 13 | SECTION 1 | 13.18 | -1.40% | 27.3 | 27.47 | 0.60% | 4 | 4.1 | 2.44% |
| | | | | | | | SECTION 2 | 13.38 | -2.94% | | | | | | |
| | | | | | | | SECTION 3 | 13.28 | -2.17% | | | | | | |
| | | | | | | | AVERAGE | 13.28 | -2.17% | | | | | | |
| 6 | 18/02/22 | 3:24 AM | 0.591 | 6142 | 1041 | 10.6 | SECTION 1 | 10.64 | -0.40% | 29.6 | 29.57 | -0.11% | 4 | 4.1 | 2.44% |
| | | | | | | | SECTION 2 | 10.74 | -1.34% | | | | | | |
| | | | | | | | SECTION 3 | 10.64 | -0.40% | | | | | | |
| | | | | | | | AVERAGE | 10.68 | -0.71% | | | | | | |
| 7 | 18/02/22 | 5:00 AM | 0.591 | 6086 | 1033 | 9.4 | SECTION 1 | 9.09 | 3.27% | 31 | 31.17 | 0.53% | 3.9 | 4.1 | 4.88% |
| | | | | | | | SECTION 2 | 8.99 | 4.33% | | | | | | |
| | | | | | | | SECTION 3 | 9.09 | 3.27% | | | | | | |
| | | | | | | | AVERAGE | 9.06 | 3.62% | | | | | | |
| 8 | 18/02/22 | 7:00 AM | 0.587 | 6037 | 1033 | 9.4 | SECTION 1 | 9.02 | 4.01% | 31 | 31.97 | 3.02% | 3.9 | 4 | 2.50% |
| | | | | | | | SECTION 2 | 9.01 | 4.12% | | | | | | |
| | | | | | | | SECTION 3 | 9.01 | 4.12% | | | | | | |
| | | | | | | | AVERAGE | 9.02 | 4.08% | | | | | | |
| 9 | 18/02/22 | 8:30 AM | 0.587 | 6032 | 1033 | 9.4 | SECTION 1 | 8.89 | 5.39% | 31 | 30.67 | -1.09% | 3.8 | 3.9 | 2.56% |
| | | | | | | | SECTION 2 | 9.09 | 3.27% | | | | | | |
| | | | | | | | SECTION 3 | 9.09 | 3.27% | | | | | | |
| | | | | | | | AVERAGE | 9.03 | 3.98% | | | | | | |
| 10 | 18/02/22 | 10:08 AM | 0.587 | 6030 | 1033 | 9.4 | SECTION 1 | 9.33 | 0.71% | 31 | 30.97 | -0.11% | 3.8 | 3.8 | 0.00% |
| | | | | | | | SECTION 2 | 9.09 | 3.27% | | | | | | |
| | | | | | | | SECTION 3 | 9.19 | 2.20% | | | | | | |
| | | | | | | | AVERAGE | 9.21 | 2.06% | | | | | | |
| 11 | 19/02/22 | 11:00 AM | 0.587 | 6029 | 1020 | 7.1 | SECTION 1 | 7.09 | 0.10% | 31 | 30.97 | -0.11% | 3.8 | 3.8 | 0.00% |
| | | | | | | | SECTION 2 | 6.89 | 2.92% | | | | | | |
| | | | | | | | SECTION 3 | 6.94 | 2.21% | | | | | | |
| | | | | | | | AVERAGE | 7.0 | 1.74% | | | | | | |

The results obtained from the previous tests were satisfactory because:

1) The records obtained by the proposed system remained within the tolerance and error margin of 15%.
2) The measurements were taken using measuring instruments for Brix, temperature, and pH, traceable to international standards (i.e. the primary measuring instruments were calibrated by international standards from the National Metrology Center).
3) The aforementioned results are reliable measurements from every monitored variable for fermentation of molasses.

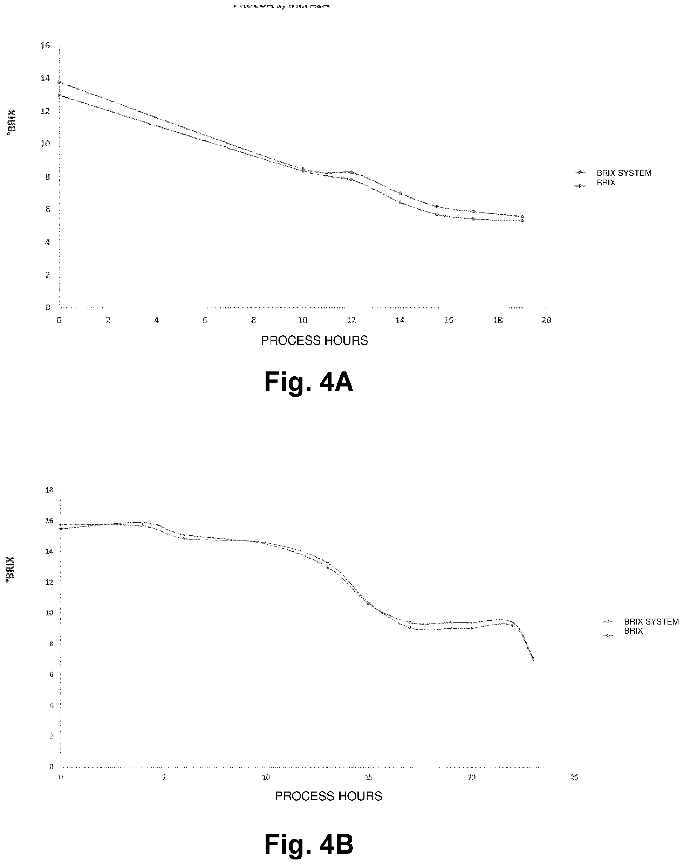

Figs. 4A and 4B show the results of the measurement of the proposed system by means of PLC vs. hydrometer. The information registered in the graphics show the behavior of Brix regarding the time of fermentation with molasses. It is then displayed that the measurement of Degrees Brix with the proposed system matches the values measured by the hydrometer. Thus, it can be concluded that the proposed monitoring system measures the Degrees Brix efficiently and correctly, within the tolerance and permissible error margin.

**Example 5: Monitoring system for alcoholic fermentation in real time in grape juice must**

The grape juice should be fermented to get wine. The test was made in a 5-day process time, in which the samples were taken every two hours, performing the corresponding measure with the hydrometer and comparing with the data displayed in the screen of the proposed monitoring system. The data is shown in table 8.

# Table 8

| | Date | Hour | Height Vega Sensor (m) | Pressure Vega Sensor (Pa) | Density by system (sensors) (kg/m3) | Brix calculated by system (°Bx) | Brix by Hydrometer (°Bx) | | % DIFFERENCE | System Temperature (°C) | Temperature T. Pattern (°C) | % DIFFERENCE | pH System | pH S. Pattern | % DIFFERENCE |
|---|---|---|---|---|---|---|---|---|---|---|---|---|---|---|---|
| | | | | | | | | | | | | | | | |
| 1 | 23/08/22 | 11:30 PM | NOT APPLICABLE | NOT APPLICABLE | NOT APPLICABLE | 21.5 | SECTION 1 | 21.16 | 1.57% | 21.5 | 21.97 | -2.17% | 4.6 | 4.6 | 0.00% |
| | | | | | | | SECTION 2 | 21.16 | 1.57% | | | | | | |
| | | | | | | | SECTION 3 | 21.16 | 1.57% | | | | | | |
| | | | | | | | AVERAGE | 21.16 | 1.57% | | | | | | |
| 2 | 24/08/22 | 2:06 AM | NOT APPLICABLE | NOT APPLICABLE | NOT APPLICABLE | 21.2 | SECTION 1 | 20.86 | 1.59% | 20.8 | 21.77 | -4.64% | 4.6 | 4.6 | 0.00% |
| | | | | | | | SECTION 2 | 20.86 | 1.59% | | | | | | |
| | | | | | | | SECTION 3 | 20.86 | 1.59% | | | | | | |
| | | | | | | | AVERAGE | 20.86 | 1.59% | | | | | | |
| 3 | 24/08/22 | 4:00 AM | NOT APPLICABLE | NOT APPLICABLE | NOT APPLICABLE | 21 | SECTION 1 | 20.66 | 1.61% | 20 | 19.97 | 0.17% | 4.6 | 4.6 | 0.00% |
| | | | | | | | SECTION 2 | 20.66 | 1.61% | | | | | | |
| | | | | | | | SECTION 3 | 20.66 | 1.61% | | | | | | |
| | | | | | | | AVERAGE | 20.66 | 1.61% | | | | | | |
| 4 | 24/08/22 | 6:00 AM | NOT APPLICABLE | NOT APPLICABLE | NOT APPLICABLE | 20.7 | SECTION 1 | 20.36 | 1.63% | 20.5 | 20.97 | -2.27% | 4.6 | 4.5 | -2.22% |
| | | | | | | | SECTION 2 | 20.36 | 1.63% | | | | | | |
| | | | | | | | SECTION 3 | 20.36 | 1.63% | | | | | | |
| | | | | | | | AVERAGE | 20.36 | 1.63% | | | | | | |
| 5 | 24/08/22 | 8:00 AM | NOT APPLICABLE | NOT APPLICABLE | NOT APPLICABLE | 20.4 | SECTION 1 | 20.16 | 1.17% | 20.8 | 20.97 | -0.80% | 4.5 | 4.5 | 0.00% |
| | | | | | | | SECTION 2 | 20.16 | 1.17% | | | | | | |
| | | | | | | | SECTION 3 | 20.16 | 1.17% | | | | | | |
| | | | | | | | AVERAGE | 20.16 | 1.17% | | | | | | |
| 6 | 24/08/22 | 10:00 AM | NOT APPLICABLE | NOT APPLICABLE | NOT APPLICABLE | 20 | SECTION 1 | 19.76 | 1.19% | 22.7 | 22.97 | -1.17% | 4.5 | 4.5 | 0.00% |
| | | | | | | | SECTION 2 | 19.76 | 1.19% | | | | | | |
| | | | | | | | SECTION 3 | 19.76 | 1.19% | | | | | | |
| | | | | | | | AVERAGE | 19.76 | 1.19% | | | | | | |
| 7 | 24/08/22 | 12:00 PM | NOT APPLICABLE | NOT APPLICABLE | NOT APPLICABLE | 19.7 | SECTION 1 | 19.36 | 1.72% | 24.1 | 23.97 | 0.56% | 4.5 | 4.5 | 0.00% |
| | | | | | | | SECTION 2 | 19.36 | 1.72% | | | | | | |
| | | | | | | | SECTION 3 | 19.36 | 1.72% | | | | | | |
| | | | | | | | AVERAGE | 19.36 | 1.72% | | | | | | |
| 8 | 24/08/22 | 02:00 PM | NOT APPLICABLE | NOT APPLICABLE | NOT APPLICABLE | 19.4 | SECTION 1 | 19.16 | 1.23% | 24.5 | 24.77% | -1.09% | 4.4 | 4.4 | 0.00% |
| | | | | | | | SECTION 2 | 19.16 | 1.23% | | | | | | |
| | | | | | | | SECTION 3 | 19.16 | 1.23% | | | | | | |
| | | | | | | | AVERAGE | 19.16 | 1.23% | | | | | | |
| 9 | 24/08/22 | 4:00 PM | NOT APPLICABLE | NOT APPLICABLE | NOT APPLICABLE | 19.2 | SECTION 1 | 18.86 | 1.76% | 25 | 25.37 | -1.46% | 4.4 | 4.4 | 0.00% |
| | | | | | | | SECTION 2 | 18.86 | 1.76% | | | | | | |
| | | | | | | | SECTION 3 | 18.86 | 1.76% | | | | | | |
| | | | | | | | AVERAGE | 18.86 | 1.76% | | | | | | |
| 10 | 24/08/22 | 6:00 PM | NOT APPLICABLE | NOT APPLICABLE | NOT APPLICABLE | 19 | SECTION 1 | 18.76 | 1.25% | 25.6 | 25.87 | -1.04% | 4.4 | 4.3 | -2.33% |
| | | | | | | | SECTION 2 | 18.76 | 1.25% | | | | | | |
| | | | | | | | SECTION 3 | 18.76 | 1.25% | | | | | | |
| | | | | | | | AVERAGE | 18.76 | 1.25% | | | | | | |
| 11 | 24/08/22 | 08:00 PM | NOT APPLICABLE | NOT APPLICABLE | NOT APPLICABLE | 18.8 | SECTION 1 | 18.46 | 1.80% | 24.9 | 24.97 | -0.27% | 4.4 | 4.3 | -2.33% |
| | | | | | | | SECTION 2 | 18.46 | 1.80% | | | | | | |
| | | | | | | | SECTION 3 | 18.46 | 1.80% | | | | | | |
| | | | | | | | AVERAGE | 18.46 | 1.80% | | | | | | |
| 12 | 24/08/22 | 10:00 PM | NOT APPLICABLE | NOT APPLICABLE | NOT APPLICABLE | 18.5 | SECTION 1 | 18.26 | 1.29% | 24.9 | 24.97 | -0.27% | 4.3 | 4.3 | 0.00% |
| | | | | | | | SECTION 2 | 18.26 | 1.29% | | | | | | |

| # | Date | Time | | | | | Section | | | | | | | | |
|---|---|---|---|---|---|---|---|---|---|---|---|---|---|---|---|
| | | | | | | | SECTION 3 | 18.26 | 1.29% | | | | | | |
| | | | | | | | AVERAGE | 18.26 | 1.29% | | | | | | |
| 13 | 25/08/22 | 12:00 AM | NOT APPLICABLE | NOT APPLICABLE | NOT APPLICABLE | 18.2 | SECTION 1 | 18.01 | 1.03% | 24.5 | 24.87 | -1.49% | 4.3 | 4.3 | 0.00% |
| | | | | | | | SECTION 2 | 18.01 | 1.03% | | | | | | |
| | | | | | | | SECTION 3 | 18.01 | 1.03% | | | | | | |
| | | | | | | | AVERAGE | 18.01 | 1.03% | | | | | | |
| 14 | 25/08/22 | 02:00 AM | NOT APPLICABLE | NOT APPLICABLE | NOT APPLICABLE | 18.06 | SECTION 1 | 17.76 | 1.65% | 24.9 | 24.47 | 1.74% | 4.3 | 4.3 | 0.00% |
| | | | | | | | SECTION 2 | 17.76 | 1.65% | | | | | | |
| | | | | | | | SECTION 3 | 17.76 | 1.65% | | | | | | |
| | | | | | | | AVERAGE | 17.76 | 1.65% | | | | | | |
| 15 | 25/08/22 | 04:00 AM | NOT APPLICABLE | NOT APPLICABLE | NOT APPLICABLE | 18 | SECTION 1 | 17.64 | 1.99% | 24.2 | 24.37 | -0.69% | 4.3 | 4.3 | 0.00% |
| | | | | | | | SECTION 2 | 17.64 | 1.99% | | | | | | |
| | | | | | | | SECTION 3 | 17.64 | 1.99% | | | | | | |
| | | | | | | | AVERAGE | 17.64 | 1.99% | | | | | | |
| 16 | 25/08/22 | 6:00 AM | NOT APPLICABLE | NOT APPLICABLE | NOT APPLICABLE | 17.8 | SECTION 1 | 17.46 | 1.90% | 24.4 | 24.47 | -0.27% | 4.2 | 4.3 | 2.33% |
| | | | | | | | SECTION 2 | 17.46 | 1.90% | | | | | | |
| | | | | | | | SECTION 3 | 17.46 | 1.90% | | | | | | |
| | | | | | | | AVERAGE | | | | | | | | |
| 17 | 25/08/22 | 8:00 AM | NOT APPLICABLE | NOT APPLICABLE | NOT APPLICABLE | 17.55 | SECTION 1 | 17.26 | 1.64% | 24 | 24.17 | -0.69% | 4.2 | 4.1 | -2.44% |
| | | | | | | | SECTION 2 | 17.26 | 1.64% | | | | | | |
| | | | | | | | SECTION 3 | 17.26 | 1.64% | | | | | | |
| | | | | | | | AVERAGE | 17.26 | 1.64% | | | | | | |
| 18 | 25/08/22 | 10:00 AM | NOT APPLICABLE | NOT APPLICABLE | NOT APPLICABLE | 17.2 | SECTION 1 | 16.86 | 1.97% | 25.6 | 25.67 | -0.26% | 4.2 | 4.1 | -2.44% |
| | | | | | | | SECTION 2 | 16.86 | 1.97% | | | | | | |
| | | | | | | | SECTION 3 | 16.86 | 1.97% | | | | | | |
| | | | | | | | AVERAGE | 16.86 | 1.97% | | | | | | |
| 19 | 25/08/22 | 12:00 PM | NOT APPLICABLE | NOT APPLICABLE | NOT APPLICABLE | 17.07 | SECTION 1 | 16.76 | 1.80% | 27 | 27.27 | -0.99% | 4.1 | 4.1 | 0.00% |
| | | | | | | | SECTION 2 | 16.76 | 1.80% | | | | | | |
| | | | | | | | SECTION 3 | 16.76 | 1.80% | | | | | | |
| | | | | | | | AVERAGE | 16.76 | 1.80% | | | | | | |
| 20 | 25/08/22 | 02:00 PM | NOT APPLICABLE | NOT APPLICABLE | NOT APPLICABLE | 17 | SECTION 1 | 16.77 | 1.34% | 28 | 28.47 | -1.66% | 4.1 | 4 | -2.50% |
| | | | | | | | SECTION 2 | 16.77 | 1.34% | | | | | | |
| | | | | | | | SECTION 3 | 16.77 | 1.34% | | | | | | |
| | | | | | | | AVERAGE | 16.77 | 1.34% | | | | | | |
| 21 | 25/08/22 | 4:00 PM | NOT APPLICABLE | NOT APPLICABLE | NOT APPLICABLE | 16.8 | SECTION 1 | 16.61 | 1.12% | 28.5 | 28.77 | -0.93% | 4.1 | 4 | -2.50% |
| | | | | | | | SECTION 2 | 16.61 | 1.12% | | | | | | |
| | | | | | | | SECTION 3 | 16.61 | 1.12% | | | | | | |
| | | | | | | | AVERAGE | 16.61 | 1.12% | | | | | | |
| 22 | 25/08/22 | 6:00 PM | NOT APPLICABLE | NOT APPLICABLE | NOT APPLICABLE | 16.5 | SECTION 1 | 16.26 | 1.44% | 29.5 | 29.97 | -1.58% | 4.1 | 4.1 | 0.00% |
| | | | | | | | SECTION 2 | 16.26 | 1.44% | | | | | | |
| | | | | | | | SECTION 3 | 16.26 | 1.44% | | | | | | |
| | | | | | | | AVERAGE | 16.26 | 1.44% | | | | | | |
| 23 | 25/08/22 | 8:00 PM | NOT APPLICABLE | NOT APPLICABLE | NOT APPLICABLE | 16.1 | SECTION 1 | 15.89 | 1.29% | 28.7 | 28.97 | -0.93% | 4.1 | 4.1 | 0.00% |
| | | | | | | | SECTION 2 | 15.89 | 1.29% | | | | | | |
| | | | | | | | SECTION 3 | 15.89 | 1.29% | | | | | | |
| | | | | | | | AVERAGE | 15.89 | 1.29% | | | | | | |
| 24 | 25/08/22 | 10:00 PM | NOT APPLICABLE | NOT APPLICABLE | NOT APPLICABLE | 15.87 | SECTION 1 | 15.66 | 1.31 | 28 | 28.37 | -1.31% | 4.1 | 4.1 | 0.00% |
| | | | | | | | SECTION 2 | 15.66 | 1.31 | | | | | | |
| | | | | | | | SECTION 3 | 15.66 | 1.31 | | | | | | |
| | | | | | | | AVERAGE | 15.66 | 1.31 | | | | | | |
| 25 | 26/08/22 | 12:00 AM | NOT APPLICABLE | NOT APPLICABLE | NOT APPLICABLE | 15.6 | SECTION 1 | 15.31 | 1.85% | 28.6 | 28.87 | -0.93% | 4.1 | 4 | -2.50% |
| | | | | | | | SECTION 2 | 15.31 | 1.85% | | | | | | |
| | | | | | | | SECTION 3 | 15.31 | 1.85% | | | | | | |

| # | Date | Time | | | | Value | Section | Sec Value | Sec % | Val | Val | % | Val | Val | % |
|---|------|------|---|---|---|-------|---------|-----------|-------|-----|-----|---|-----|-----|---|
| | | | | | | | AVERAGE | 15.31 | 1.85% | | | | | | |
| 26 | 26/08/22 | 02:00 AM | NOT APPLICABLE | NOT APPLICABLE | NOT APPLICABLE | 15.19 | SECTION 1 | 14.86 | 2.16% | 29 | 29.17 | -0.57% | 4 | 4 | 0.00% |
| | | | | | | | SECTION 2 | 14.86 | 2.16% | | | | | | |
| | | | | | | | SECTION 3 | 14.86 | 2.16% | | | | | | |
| | | | | | | | AVERAGE | 14.86 | 2.16% | | | | | | |
| 27 | 26/08/22 | 04:00 AM | NOT APPLICABLE | NOT APPLICABLE | NOT APPLICABLE | 14.6 | SECTION 1 | 14.16 | 3.00% | 28.5 | 28.67 | -0.58% | 4 | 4 | 0.00% |
| | | | | | | | SECTION 2 | 14.16 | 3.00% | | | | | | |
| | | | | | | | SECTION 3 | 14.16 | 3.00% | | | | | | |
| | | | | | | | AVERAGE | 14.16 | 3.00% | | | | | | |
| 28 | 26/08/22 | 6:00 AM | NOT APPLICABLE | NOT APPLICABLE | NOT APPLICABLE | 13.9 | SECTION 1 | 13.66 | 1.71% | 29.5 | 29.37 | 0.45% | 3.4 | 3.4 | 0.00% |
| | | | | | | | SECTION 2 | 13.66 | 1.71% | | | | | | |
| | | | | | | | SECTION 3 | 13.66 | 1.71% | | | | | | |
| | | | | | | | AVERAGE | 13.66 | 1.71% | | | | | | |
| 29 | 26/08/22 | 8:00 AM | NOT APPLICABLE | NOT APPLICABLE | NOT APPLICABLE | 12.6 | SECTION 1 | 12.26 | 2.68% | 30.2 | 30.47 | -0.88% | 3.5 | 3.5 | 0.00% |
| | | | | | | | SECTION 2 | 12.26 | 2.68% | | | | | | |
| | | | | | | | SECTION 3 | 12.26 | 2.68% | | | | | | |
| | | | | | | | AVERAGE | 12.26 | 2.68% | | | | | | |
| 30 | 26/08/22 | 10:00 AM | NOT APPLICABLE | NOT APPLICABLE | NOT APPLICABLE | 12.3 | SECTION 1 | 12.11 | 1.53% | 32 | 32.37 | -1.14% | 3.4 | 3.2 | -6.25% |
| | | | | | | | SECTION 2 | 12.11 | 1.53% | | | | | | |
| | | | | | | | SECTION 3 | 12.11 | 1.53% | | | | | | |
| | | | | | | | AVERAGE | 12.11 | 1.53% | | | | | | |
| 31 | 26/08/22 | 12:00 PM | NOT APPLICABLE | NOT APPLICABLE | NOT APPLICABLE | 12.17 | SECTION 1 | 11.96 | 1.71% | 32.4 | 32.47 | -0.20% | 3.2 | 3.2 | 0.00% |
| | | | | | | | SECTION 2 | 11.96 | 1.71% | | | | | | |
| | | | | | | | SECTION 3 | 11.96 | 1.71% | | | | | | |
| | | | | | | | AVERAGE | 11.96 | 1.71% | | | | | | |
| 32 | 26/08/22 | 02:00 PM | NOT APPLICABLE | NOT APPLICABLE | NOT APPLICABLE | 11.7 | SECTION 1 | 11.36 | 2.89% | 32.5 | 32.67 | -0.51% | 3 | 3 | 0.00% |
| | | | | | | | SECTION 2 | 11.36 | 2.89% | | | | | | |
| | | | | | | | SECTION 3 | 11.36 | 2.89% | | | | | | |
| | | | | | | | AVERAGE | 11.36 | 2.89% | | | | | | |
| 33 | 26/08/22 | 4:00 PM | NOT APPLICABLE | NOT APPLICABLE | NOT APPLICABLE | 10.4 | SECTION 1 | 10.09 | 2.96% | 34 | 34.47 | -1.37% | 3.35 | 3.3 | -1.52% |
| | | | | | | | SECTION 2 | 10.09 | 2.96% | | | | | | |
| | | | | | | | SECTION 3 | 10.09 | 2.96% | | | | | | |
| | | | | | | | AVERAGE | 10.09 | 2.96% | | | | | | |
| 34 | 26/08/22 | 6:00 PM | NOT APPLICABLE | NOT APPLICABLE | NOT APPLICABLE | 9.4 | SECTION 1 | 9.14 | 2.73% | 34 | 34.27 | -0.78% | 3.35 | 3.3 | -1.52% |
| | | | | | | | SECTION 2 | 9.14 | 2.73% | | | | | | |
| | | | | | | | SECTION 3 | 9.14 | 2.73% | | | | | | |
| | | | | | | | AVERAGE | 9.14 | 2.73% | | | | | | |
| 35 | 26/08/22 | 8:00 PM | NOT APPLICABLE | NOT APPLICABLE | NOT APPLICABLE | 9.33 | SECTION 1 | 8.89 | 4.68% | 33.4 | 33.47 | -0.20% | 3 | 3 | 0.00% |
| | | | | | | | SECTION 2 | 8.89 | 4.68% | | | | | | |
| | | | | | | | SECTION 3 | 8.89 | 4.68% | | | | | | |
| | | | | | | | AVERAGE | 8.89 | 4.68% | | | | | | |
| 36 | 27/08/22 | 07:00 AM | NOT APPLICABLE | NOT APPLICABLE | NOT APPLICABLE | 4.88 | SECTION 1 | 4.63 | 5.06% | 30 | 30.67 | -2.22% | 3.3 | 3.2 | -3.12% |
| | | | | | | | SECTION 2 | 4.63 | 5.06% | | | | | | |
| | | | | | | | SECTION 3 | 4.63 | 5.06% | | | | | | |
| | | | | | | | AVERAGE | 4.63 | 5.06% | | | | | | |
| 37 | 27/08/22 | 02:00 PM | NOT APPLICABLE | NOT APPLICABLE | NOT APPLICABLE | 4.7 | SECTION 1 | 4.44 | 5.47% | 30.7 | 30.87 | -0.54% | 3.2 | 3.2 | 0.00% |
| | | | | | | | SECTION 2 | 4.44 | 5.47% | | | | | | |
| | | | | | | | SECTION 3 | 4.44 | 5.47% | | | | | | |
| | | | | | | | AVERAGE | 4.44 | 5.47% | | | | | | |
| 38 | 27/08/22 | 04:00 PM | NOT APPLICABLE | NOT APPLICABLE | NOT APPLICABLE | 4.3 | SECTION 1 | 3.99 | 7.14% | 30 | 30.27 | -0.89% | 3 | 3 | 0.00% |
| | | | | | | | SECTION 2 | 3.99 | 7.14% | | | | | | |
| | | | | | | | SECTION 3 | 3.99 | 7.14% | | | | | | |
| | | | | | | | AVERAGE | 3.99 | 7.14% | | | | | | |

| 39 | 27/08/22 | 06:00 PM | NOT APPLICABLE | NOT APPLICABLE | NOT APPLICABLE | 2.4 | SECTION 1 | 2.15 | 10.29% | 27 | 27.17 | -0.61% | 3 | 3 | 0.00% |
|---|---|---|---|---|---|---|---|---|---|---|---|---|---|---|---|
| | | | | | | | SECTION 2 | 2.15 | 10.29% | | | | | | |
| | | | | | | | SECTION 3 | 2.15 | 10.29% | | | | | | |
| | | | | | | | AVERAGE | 2.15 | 10.29% | | | | | | |

The results obtained from the previous tests were satisfactory because:

1) The records obtained by the proposed system remained within the tolerance and error margin of 15%.
2) The measurements were taken using measuring instruments for Brix, temperature, and pH, traceable to international standards (i.e. the primary measuring instruments were calibrated by international standards from the National Metrology Center).
3) The aforementioned results are reliable measurements from every monitored variable for fermentation of grape juice.

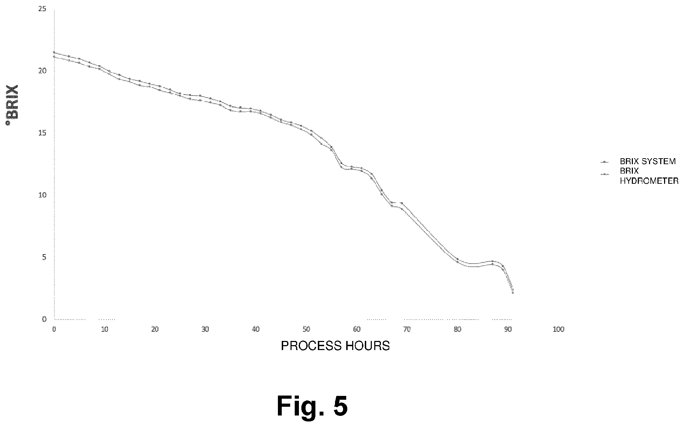

Fig. 5 shows the results of proposed Brix system by means of PLC vs. hydrometer. The information recorded in the graphics shows the behavior of Brix regarding the time of fermentation with grape juice. The displays shows that the measuring of the Degrees Brix with the proposed system matches the values measured by the hydrometer. Therefore, it can be concluded that the proposed monitoring system measures Degrees Brix efficiently and correctly, within the tolerance and permissible error margin.

The scope of the present invention is defined in the following set of claims.

## Claims

1. A method for monitoring the fermentation of alcoholic beverages, comprising:

   carrying out an alcoholic fermentation process of a must to be fermented inside a fermentation tank (1);
   acquiring, by a plurality of sensors (2-5) arranged within the fermentation tank (1), different data of the must, said acquired data comprising: level data, pressure, temperature, and pH;
   receiving, by a computing device (6), the acquired data and computing a density of the must by implementing the following equation using the acquired data: $\rho = \dfrac{P}{gh}$ , where $\rho$ is the density; P is the pressure; g is the specific gravity; and h is the height of the must; and
   computing, by the computing device (6), the Degrees Brix of the must as a function of the computed density.

2. The method of claim 1, wherein the computing device (6) further comprises:

   computing the alcohol volume percentage of the must using the computed Degrees Brix; and
   computing the fermentation efficiency percentage of the must using the computed alcohol volume percentage.

3. The method of claim 1 or 2, wherein the must to be fermented is of dark color and/or is murky.

4. The method of any one of the previous claims, wherein the must to be fermented is selected from barley, molasses, grape juice or agave juice.

5. The method of claim 1, further comprising displaying the computed Degrees Brix on a user interface (8) and/or on a computer application (11) operatively connected with the computed device (6).

6. The method of claim 2, further comprising displaying the computed fermentation efficiency and the alcohol volume percentage on a user interface (8) and/or on a computer application (11) operatively connected with the computed device (6).

7. The method of claim 2, wherein the computed Degrees Brix, the fermentation efficiency and the alcohol volume percentage are stored in a cloud computing structure (10).

8. The method of any one of the previous claims, further comprising raising a warning signal or an alarm when the computed Degrees Brix, the fermentation efficiency and/or the alcohol volume percentage are outside a given permissible range.

9. A system for monitoring the fermentation of alcoholic beverages, comprising:

a fermentation tank (1) at which an alcoholic fermentation process of a must to be fermented takes place;
a plurality of sensors arranged within said tank (1) to acquire different data of the alcoholic fermentation process, said plurality of sensors comprising a radar sensor (2), a temperature sensor (3); a pH sensor (4) and a pressure sensor (5), and said acquired data comprising: level data, pressure, temperature, and pH; and
a computing device (6), configured to:

receive the acquired data,

compute a density of the must by implementing the following equation using the acquired data: $\rho = \dfrac{P}{gh}$
, where $\rho$ is the density; P is the pressure; g is the specific gravity; and h is the height of the must; and
compute the Degrees Brix of the must as a function of the computed density.

10. The system of claim 9, wherein the computing device (6) is further configured to compute the alcohol volume percentage of the must using the computed Degrees Brix and to compute the fermentation efficiency percentage of the must using the computed alcohol volume percentage.

11. The system of claim 9 or 10, wherein the plurality of sensors are arranged at different positions within the fermentation tank (1).

12. The system of claim 9, 10 or 11, further comprising a user interface and/or a computer application (11) operatively connected to the computer device (6) and configured to at least display the computed Degrees Brix.

13. The system of any one of the previous claims 9 to 12, further comprising a cloud computing structure (10) to at least store the computed Degrees Brix.

**Fig. 1**

**Fig. 2A**

**Fig. 2B**

**Fig. 3A**

**Fig. 3B**

Fig. 4A

Fig. 4B

Fig. 5

Europäisches
Patentamt

European
Patent Office

Office européen
des brevets

# EUROPEAN SEARCH REPORT

Application Number

EP 23 17 4146

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ES 2 375 773 A1 (LETE ALDASORO JUAN MANUEL [ES]) 6 March 2012 (2012-03-06) | 1-8 | INV.<br>C12G1/02 |
| Y | * the whole document * | 9-13 | C12G3/02<br>C12G3/021 |
| Y | US 2022/252491 A1 (CHABOT MARC-ANDRE [CA] ET AL) 11 August 2022 (2022-08-11) * the whole document * | 9-13 | G01N33/14<br>C12C11/00 |
| A | US 2020/157479 A1 (NUNES NOGUEIRA ROGÉRIO [PT] ET AL) 21 May 2020 (2020-05-21) * the whole document * | 1-13 | |
| A | Anonymous: "Brix", Wikipedia, 4 January 2017 (2017-01-04), pages 1-5, XP055346004, Retrieved from the Internet: URL:https://en.wikipedia.org/wikiBrix?oldid=758277966 [retrieved on 2017-02-15] * the whole document * | 1-13 | |
| A | EP 0 947 821 A1 (PLASMATI EUSTACHIO [IT]) 6 October 1999 (1999-10-06) * the whole document * | 1-13 | TECHNICAL FIELDS SEARCHED (IPC)<br><br>C12G<br>G01N<br>C12C |
| A | US 2018/072972 A1 (SHIN JONG-WOOK [US] ET AL) 15 March 2018 (2018-03-15) * the whole document * | 1-13 | |
| A | US 4 659 662 A (HSU WIN-PEN [US]) 21 April 1987 (1987-04-21) * the whole document * | 1-13 | |

The present search report has been drawn up for all claims

| Place of search | Date of completion of the search | Examiner |
|---|---|---|
| The Hague | 10 October 2023 | Fiorenza, Francesca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 17 4146

This annex lists the patent family members relating to the patent documents cited in the above-mentioned European search report.
The members are as contained in the European Patent Office EDP file on
The European Patent Office is in no way liable for these particulars which are merely given for the purpose of information.

10-10-2023

| Patent document cited in search report | | Publication date | Patent family member(s) | | Publication date |
|---|---|---|---|---|---|
| ES 2375773 | A1 | 06-03-2012 | NONE | | |
| US 2022252491 | A1 | 11-08-2022 | CA | 3148301 A1 | 10-08-2022 |
| | | | US | 2022252491 A1 | 11-08-2022 |
| US 2020157479 | A1 | 21-05-2020 | AU | 2019383774 A1 | 10-06-2021 |
| | | | AU | 2023202268 A1 | 04-05-2023 |
| | | | EP | 3884029 A1 | 29-09-2021 |
| | | | US | 2020157479 A1 | 21-05-2020 |
| | | | WO | 2020104843 A1 | 28-05-2020 |
| EP 0947821 | A1 | 06-10-1999 | EP | 0947821 A1 | 06-10-1999 |
| | | | IT | MI980663 A1 | 30-09-1999 |
| US 2018072972 | A1 | 15-03-2018 | US | 2018072972 A1 | 15-03-2018 |
| | | | WO | 2018049342 A1 | 15-03-2018 |
| US 4659662 | A | 21-04-1987 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120269925 A1
- WO 2020012459 A1
- EP 3763828 A1
- US 20200292501 A1